# EUROPEAN PATENT APPLICATION

(11) **EP 3 604 276 A1**
(43) Date of publication of application: **05.02.2020**
(21) Application number: 18186648.4
(22) Date of filing: 31.07.2018
(51) Int. Cl.: C07C 317/04, H01M 10/0567, H01M 10/0525, H01M 10/0568, H01M 10/0569

(54) **NEW COMPONENTS FOR ELECTROLYTE COMPOSITIONS**

(71) Applicant: SOLVAY SA, 1120 Brussels (BE)
(72) Inventor: BUISINE, Olivier, 69230 Saint Genis-Lasal (FR); METZ, François, 69540 Irigny (FR); JAUNZEMS, Janis, 30559 Hannover (DE); KIM, Yeon-Joon, Songpa-gu, Seoul (KR)
(74) Representative: Mross, Stefan P.M.

(57) **Abstract**

The present invention relates to the field of chemical components for electrolyte compositions which are useful in electrochemical cells, such as lithium-ion batteries.

More specifically, the invention provides new components and their combinations, which can be used as solvents, additives and/or electrolyte salts. These components are suitable to improve various characteristics of electrolyte compositions and, *in fine,* of the electrochemical cells in which said electrolyte compositions are incorporated. The invention also relates to the manufacturing processes of these components.

## Description

### TECHNICAL FIELD

The present invention relates to the field of chemical components for electrolyte compositions which are useful in electrochemical cells, such as lithium-ion batteries.

More specifically, the invention provides new solvents, additives and/or electrolyte salts and their combinations, which are suitable to improve various characteristics of electrolyte compositions and, *in fine*, of the electrochemical cells in which said electrolyte compositions are incorporated.

### BACKGROUND ART

Among the electrochemical cells, lithium-ion batteries are rechargeable batteries that are commonly used in various devices, such as portable home electronics, energy storage systems or electric vehicles. Depending on their final usage, the expectations of lithium-ion batteries in terms of safety, performance and cost are more and more challenging. Various approaches have been investigated to overcome the limitations of commonly used lithium-ion batteries. For example, new electrode materials have been developed to improve capacity. Another approach has and still consists in formulating electrolyte compositions with specific chemical solvents, additives and electrolyte salts to improve various characteristics of the battery such as cycling performance, reversible capacity and bulging limitation.

Despite the efforts in the art, we believe that there is still room for improvement for providing electrolyte compositions ingredients capable of fulfilling the required specifications of electrolyte compositions, especially those intended to be used in lithium-ion batteries.

Especially, at cathode potentials above about 4.35 V, the electrolyte solvents can decompose, which can result in a loss of battery performance. Electrolyte decomposition can also occur, generating gas which can cause swelling of the battery. There remains a need for an electrolyte composition that, when used in a lithium ion battery, can exhibit high cycle performance at low and high temperature, storage performance at high temperature, and power at low temperature.

### BRIEF DESCRIPTION OF THE INVENTION

The above technical problems are solved thanks to the invention such as recited in the claims. The Applicant discovered new chemical components and/or new combinations of chemical components that are useful ingredients for electrolyte compositions intended to be used in electrochemical cells, especially in lithium ion batteries. The chemical components according to the present invention can be used as solvents, additives or electrolyte salts depending on their chemical structure and their amount in the electrolyte composition. They can notably improve various performance characteristics of an electrolyte composition to be used in an electrochemical cell, such as quality of the solid electrolyte interphase (SEI) that will forms on the electrodes surface in use, chemical stability, ionic conductivity, thermal stability, reversible capacity, cycle characteristics and gas generation.

### DEFINITIONS

In the present disclosure, the expression "ranging from ... to ..." should be understood has including the limits.

The term "electrolyte composition" as used herein, refers to a non-aqueous liquid chemical composition suitable for use as an electrolyte in an electrochemical cell.

The term "solvent" in connection with said electrolyte composition typically refers to a compound that is present in the electrolyte composition in an amount of at least 20% wt relative to the total weight of the electrolyte composition.

The term "additive" in connection with said electrolyte composition typically refers to a compound that is present in the electrolyte composition in an amount of less than 20% wt relative to the total weight of the electrolyte composition.

The term "electrolyte salt" as used herein, refers to an ionic salt that is at least partially soluble in the electrolyte composition and that at least partially dissociates into ions in the electrolyte composition to form a conductive electrolyte composition.

An "electrolyte solvent" as defined herein is a solvent or a solvent mixture for an electrolyte composition.

The term "alkyl" refers to a linear or branched, saturated or unsaturated, substituted or unsubstituted, hydrocarbon chain, which comprises or not heteroatoms such as P, B, N, O, and/or S. For illustrative purposes and without being exhaustive, as possible substituents, mention can be made of halogens, alkyl, cycloalkyl, aryl, heteroaryl and/or heterocyclyl groups. A cycloalkyl group may contain up to 8 carbon atoms. An aryl group may be a monocyclic or bicyclic aromatic group. The aryl group may contain from 5 to 12 carbon atoms. A heteroaryl group may be a monocyclic or bicyclic group. The heteroaryl group may contain from 1 to 12 carbon atoms and one or more N, O or S atoms. The heteroaryl group may be a 5 or 6-membered ring containing one or more N atoms. A heterocyclyl group may be a monocyclic or bicyclic group. The heterocyclyl group may contain from 1 to 12 carbon atoms and one or more N, O or S atoms. According to an embodiment, said "alkyl" group is linear. According to an embodiment, said "alkyl" group is a C₁ to C₄ alkyl group. According to an embodiment, said "alkyl" group is saturated. According to an embodiment, said "alkyl" group is unsubstituted. According to an embodiment, said "alkyl" group does not comprise heteroatoms. Any of these embodiments can be combined with one another. Preferably "alkyl" means a saturated, unsubstituted group comprising only carbon and hydrogen atoms, preferably 1 to 4 carbon atoms.

The term "halogenoalkyl" specifically refers to an alkyl group as defined above comprising at least one halogen atom. Especially the term "fluoroalkyl" refers to an alkyl group comprising at least one fluorine atom. The term "perhalogenoalkyl" refers to an alkyl group comprising only halogen atoms, in addition to the carbon atoms, and devoid of hydrogen atoms. The term "perfluoroalkyl" especially refers to an alkyl group comprising only fluorine atoms, in addition to the carbon atoms, and devoid of hydrogen atoms.

The term "halogenoalkoxy" specifically refers to an alkoxy group (well known to those skilled in the art) wherein at least one hydrogen atom is substituted by a halogen atom. The term "fluoroalkoxy" especially refers to an alkoxy group wherein at least one hydrogen atom is substituted by fluorine.

The term "reaction medium" refers to the medium in which the reaction takes place. The reaction medium comprises the reaction solvent when the reaction is performed in a solvent, the catalyst when a catalyst is used, and, depending on the progression of the reaction, the reactants and/or the products of the reaction. In addition, it can comprise additives and impurities.

The term "anode" refers to the electrode of an electrochemical cell, at which oxidation occurs. In a secondary (i.e. rechargeable) battery, the anode is the electrode at which oxidation occurs during discharge and reduction occurs during charging.

The term "cathode" refers to the electrode of an electrochemical cell, at which reduction occurs. In a secondary (i.e. rechargeable) battery, the cathode is the electrode at which reduction occurs during discharge and oxidation occurs during charging.

The term "lithium ion battery" refers to a type of rechargeable battery in which lithium ions move from the anode to the cathode during discharge and from the cathode to the anode during charge.

The equilibrium potential between lithium and lithium ion is the potential of a reference electrode using lithium metal in contact with the non-aqueous electrolyte containing lithium salt at a concentration sufficient to give about 1 mole/liter of lithium ion concentration, and subjected to sufficiently small currents so that the potential of the reference electrode is not significantly altered from its equilibrium value (Li/Li⁺). The potential of such a Li/Li⁺ reference electrode is assigned here the value of 0.0V. Potential of an anode or cathode means the potential difference between the anode or cathode and that of a Li/Li⁺ reference electrode. Herein voltage means the voltage difference between the cathode and the anode of a cell, neither electrode of which may be operating at a potential of 0.0V.

An "energy storage device" is a device that is designed to provide electrical energy on demand, such as a battery or a capacitor. Energy storage devices contemplated herein at least in part provide energy from electrochemical sources.

The term "SEI", as used herein, refers to a solid electrolyte interphase layer formed on the active material of an electrode. A lithium-ion secondary electrochemical cell is assembled in an uncharged state and must be charged (a process called formation) for use. During the first few charging events (battery formation) of a lithium-ion secondary electrochemical cell, components of the electrolyte are reduced or otherwise decomposed or incorporated onto the surface of the negative active material and oxidized or otherwise decomposed or incorporated onto the surface of the positive active material, electrochemically forming a solid-electrolyte interphase on the active materials. These layers, which are electrically insulating but ionically conducting, help prevent decomposition of the electrolyte and can extend the cycle life and improve the performance of the battery. On the anode, the SEI can suppress the reductive decomposition of the electrolyte; on the cathode, the SEI can suppress the oxidation of the electrolyte components.

### DESCRIPTION OF THE INVENTION

One subject matter of the invention is a compound of formula (VI) 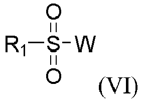 wherein R₁ denotes H, a halogen atom, an alkyl group or an halogenoalkyl group; and W denotes a group selected from groups (A), (B) and (C) 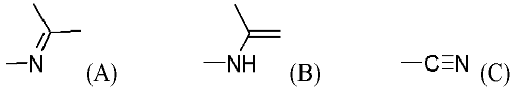 provided that R₁ is different from -CH₃ when W is (A) or (C).

In one embodiment (and still provided that R₁ is different from -CH₃ when W is (A) or (C)), R₁ denotes H or an alkyl group, preferably a C₁ to C₄ alkyl group, more preferably a C₁ to C₃ alkyl group, a C₁ to C₂ alkyl group, especially a C₁ alkyl group. In one sub-embodiment wherein W is (B), R₁ denotes CH₃.

In one alternative embodiment, R₁ denotes a halogen atom being preferably fluorine, or a halogenoalkyl group being preferably a fluoroalkyl group. R₁ denotes more preferably a halogenoalkyl group, still more preferably a C₁ to C₄ halogenoalkyl group and even more preferably a C₁ to C₄ fluoroalkyl group; the latter can be perfluorinated. More preferably, R₁ denotes a C₁ to C₃ fluoro- or perfluoroalkyl group, still more preferably a C₁ to C₂ fluoro- or perfluoroalkyl group. R₁ can especially be selected from: -CH₂F, -CHF₂, -CF₃, -CH₂-CH₂F, -CH₂-CHF₂, -CH₂-CF₃, -CHF-CH₃, -CHF-CH₂F, -CHF-CHF₂, -CHF-CF₃, -CF₂-CH₃, -CF₂-CH₂F, -CF₂-CHF₂, and -CF₂-CF₃. In a sub-embodiment, R₁ is a C₁ fluoro- or perfluoroalkyl group. It can especially be selected from -CH₂F, -CHF₂, and -CF₃. In one sub-embodiment R₁ is -CF₃. In another sub-embodiment R₁ is -CHF₂. In one sub-embodiment, R₁ is -CH₂F.

In one embodiment, which can be combined with any of the previous ones concerning the choice of R₁, W is (A) or (B). More preferably, W is (A) or (B) and R₁ denotes a halogen atom or a C₁ to C₄ fluoro- or perfluoroalkyl group, preferably a C₁ to C₃ fluoro or perfluoroalkyl group, more preferably a C₁ to C₂ fluoro or perfluoroalkyl group, even more preferably a -CF3 group.

In one alternative embodiment, which can be combined with any of the previous ones concerning the choice of R₁, W is (C). More preferably, W is (C) and R₁ denotes a halogen atom or a C₁ to C₄ fluoro- or perfluoroalkyl group, preferably a C₁ to C₃ fluoro or perfluoroalkyl group, more preferably a C₁ to C₂ fluoro or perfluoroalkyl group, even more preferably a -CF3 group.

Preferred compounds of formula (VI) are given in table 6. Among these compounds, compounds (VI).1, (VI).5 and (VI).9 are particularly preferred.

**Table 1: preferred compounds of formula (VI)**

| | R₁ | W |
|---|---|---|
| (VI).1 | CF₃ | A |
| (VI).2 | CHF₂ | A |
| (VI).3 | CH₂F | A |
| (VI).4 | F | A |
| (VI).5 | CF₃ | B |
| (VI).6 | CHF₂ | B |
| (VI).7 | CH₂F | B |
| (VI).8 | F | B |
| (VI).9 | CF₃ | C |
| (VI).10 | CHF₂ | C |
| (VI).11 | CH₂F | C |
| (VI).12 | F | C |

One subject matter of the invention is a process for making a compound of formula (VI) 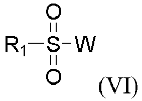 wherein R₁ denotes H, a halogen atom, an alkyl group or an halogenoalkyl group; and W denotes a group selected from groups (A), (B) and (C) 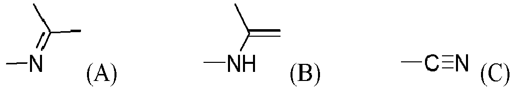 which comprises:
- either a step (1) of reacting a compound of formula (i₅) 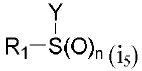 wherein R₁ denotes H, a halogen atom, an alkyl group or an halogenoalkyl group; Y denotes a halogen atom; and n is an integer equal to 1 or 2;
   with a compound of formula (iv) 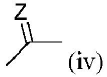 wherein Z denotes NH or NOH,
- either a step (Ibis) of reacting a compound of formula (i₅) 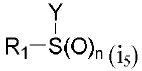 wherein R₁ denotes H, a halogen atom, an alkyl group or an halogenoalkyl group; Y denotes a-NH₂ group; and n is an integer equal to 2;
   with a compound of formula (iv) 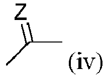 wherein Z denotes O,
- either a step (Iter) of reacting a compound of formula (i5) 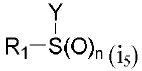 wherein R₁ denotes H, a halogen atom, an alkyl group or an halogenoalkyl group; Y denotes a halogen atom; and n is an integer equal to 2;
   with a compound of formula (iv') 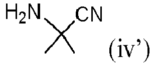 so as to form a compound of formula 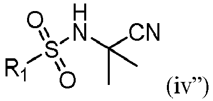 and then react compound (iv") with a base
- either a step (1quater) of reacting a compound of formula (i5) 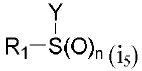
   wherein R₁ denotes H, a halogen atom, an alkyl group or an halogenoalkyl group; Y denotes a halogen atom; and n is an integer equal to 2;
   with a cyanide compound of formula M'C=N wherein M' is selected from H and a trialkylsilyl group, or a salt thereof,
- or a step (1quinquies) of reacting a compound of formula (i5) 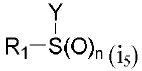
   wherein R₁ denotes H, a halogen atom, an alkyl group or an halogenoalkyl group; Y denotes a-ORₘ group wherein Rₘ is an alkali metal; and n is an integer equal to 1;
   with a cyanide compound of formula M'C=N wherein M' is an halogen atom.
   provided that R₁ is different from -CH₃ when Z is NOH, Y is Cl and n is equal to 1 or when M is Cl, Y is OK and n is equal to 1.

In one embodiment, the process for making compounds (VI) is more specifically a process for making compound(s) of formula (VIA) and/or (VIB) 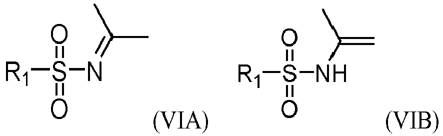
wherein R₁ denotes H, a halogen atom, an alkyl group or a halogenoalkyl group; preferably H, a C₁ to C₄ alkyl group, a halogen atom, or a C₁ to C₄ fluoroalkyl group. This process comprises either the step (1), either the step (Ibis) or the step (Iter) of the general process for making compounds of formula (VI),
provided that R₁ is different from -CH₃ when Z is NOH, Y is -Cl and n is equal to 1.

In another embodiment, the process for making compounds (VI) is more specifically a process for making compound of formula (VIC) 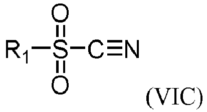
wherein R₁ denotes H, a halogen atom, an alkyl group or a halogenoalkyl group; preferably R₁ denotes hydrogen, a C₁ to C₄ alkyl group, a halogen atom, or a C₁ to C₄ fluoroalkyl group. This process therefore comprises either the step (1quater) or the step (1 quinquies) of the general process for making compounds of formula (VI) described above,
provided that R₁ is different from CH₃ when M is Cl, Y is OK and n is equal to 1.

Each of the alternative steps (1) to (1quinquies) will now be described in details.

For each of these, R₁ in compound of formula (i₅) preferably denotes hydrogen, a C₂ to C₄ alkyl group, a halogen atom, or a C₁ to C₄ fluoroalkyl group. R₁ can more particularly be selected from hydrogen or a C₂ to C₄ alkyl group. Alternatively, R₁ can more particularly be selected from a halogen atom or a C₁ to C₄ fluoroalkyl group; the latter can be perfluorinated. More preferably, R₁ denotes a C₁ to C₃ fluoro- or perfluoroalkyl group, still more preferably a C₁ to C₂ fluoro- or perfluoroalkyl group. R₁ can especially be selected from: -CH₂F, -CHF₂, -CF₃, -CH₂-CH₂F, -CH₂-CHF₂, -CH₂-CF₃, -CHF-CH₃, -CHF-CH₂F, -CHF-CHF₂, -CHF-CF₃, -CF₂-CH₃, -CF₂-CH₂F, -CF₂-CHF₂, and -CF₂-CF₃. In a sub-embodiment, R₁ is a C₁ fluoro- or perfluoroalkyl group. It can especially be selected from -CH₂F, -CHF₂, and -CF₃. In one sub-embodiment R₁ is -CF₃. In another sub-embodiment R₁ is -CHF₂. In another sub-embodiment R₁ is -CH₂F.

### Step (1)

Referring to compound of formula (i₅) used in step (1), among possible halogen atoms denoted by Y, chlorine is preferred. In one sub-embodiment, compound of formula (i₅) used in step (1) is such as n is equal to 1 and compound of formula (iv) used in step (1) is such as Z denotes NOH. In an alternative sub-embodiment compound of formula (i₅) used in step (1) is such as n is equal to 2 and compound of formula (iv) used in step (1) is such as Z denotes NH. Both of these sub-embodiments enable the formation of compound (VI) wherein W denotes the group A.

The initial molar ratio (compound of formula (i₅)/compound of formula (iv)) preferably ranges from 0.70 to 1.40, preferably from 0.80 to 1.20.

A solvent can be used as reaction medium to perform the reaction between compound of formula (i₅) with compound of formula (iv). An organic solvent, preferably an aprotic one, can advantageously be used. Mention can be made of chlorinated solvents, such as dichloromethane, chloroform, o-dichlorobenzene, being preferably dichloromethane. The reaction can alternatively be performed without solvent. In this case, one or both of the reactants can serve as reaction medium.

A hydrogen halide by-product may be formed during the reaction between compound (i₅) and compound (iv). To avoid that the hydrogen halide inhibits the reaction by decreasing pH or protonates the reactants, a base can be added in the reaction medium. As suitable base, mention can be made of organic bases, especially trialkylamines, and pyridines, and inorganic bases, especially those selected from alkali metals and alkaline earth metals carbonates such as Na₂CO₃, K₂CO₃, being preferably a trialkylamine and more preferably triethylamine. The amount of base depends of the one of compound (i₅); the molar ratio (base/compound (i₅)) preferably ranges from 1:1 to 1.2:1. In contact with the hydrogen halide, the base will form the corresponding hydrohalide (for instance, triethylamine will form triethylamine hydrohalide). It can be easily removed from the reaction medium by known methods, for instance by liquid-liquid extraction. Hydrogen halide can be eliminated by stripping.

The reaction step between compound of formula (i₅) with compound of formula (iv) can be performed at a temperature ranging from 5°C to 50°C, preferably from 10°C to 30°C. It can advantageously be performed at ambient temperature (20°C to 25°C). It is advantageous to perform this reaction step in two stages, by first contacting compound (i₅) with compound of formula (iv) (and optionally the base) at a temperature ranging from -5°C to 10°C, preferably from 0°C to 5°C, and preferably by maintaining the temperature below 10°C until completion of this contacting stage, and then by raising the temperature to a temperature ranging from 15°C to 50°C, preferably from 20°C to 25°C for the time necessary to complete the reaction.

The reaction is preferably performed at atmospheric pressure.

Reaction time can vary widely as a function of the reaction temperature chosen. It can range from 1 hour to one day, especially from 1 hour to 12 hours, more particularly from 1 hour to 5 hours.

The progress of the reaction can be monitored by the degree of conversion of the compound of formula (i₅), which is the molar ratio of the amount of compound of formula (i₅) which has been consumed to the initial amount of compound of formula (i₅) in the reaction medium, this degree being readily calculated after dosing compound of formula (i₅) remaining in the reaction medium.

The reaction medium can then be treated in a way known *per se* in order to separate the different compounds present, especially to isolate the compound of formula (VI) obtained. It makes it possible for the remaining starting materials to be recycled in order to produce an additional amount of the targeted compound of formula (VI). One or more liquid/solid separation operations can be carried out, for example in order to separate possible solid impurities from the reaction medium. The techniques used can be crystallization, filtration on different types of supports, centrifugation, separation on settling and evaporation, this list not being exhaustive. Alternatively or in addition, one or more liquid/liquid separation operations can be carried out in order to separate and/or purify the product obtained. The techniques used can be distillation, liquid/liquid extraction, separation by reverse osmosis or separation by ion-exchange resins, this list not being exhaustive. These liquid/solid and liquid/liquid separation operations can be carried out under continuous or batch conditions, it being possible for a person skilled in the art to choose the most appropriate conditions.

Accordingly, the process for making compound of formula (VI) can additionally comprise a step which consists in isolating said compound (VI), for instance by performing at least one liquid/solid separation and/or at least one liquid/liquid separation. In one specific embodiment, said at least one liquid/solid separation is a filtration and said at least one liquid/liquid separation is a distillation.

Preferably, isolated compound of formula (VI) has a purity degree of at least 94% wt, 95% wt, 96% wt, 97% wt, 98 % wt, or even 99 % wt.The process for making compound of formula (VI) can additionally comprise a step which consists in separating part or all of the unreacted compound of formula (i₅), (vi) and in recycling these compounds in the process.

Compound of formula (i₅) wherein n is equal to 1 can advantageously be obtained by a reaction step between a compound of formula (i₆) 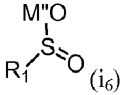
wherein R₁ such as described above in connection with compound of formula (i₅) and M" denotes an alkali metal,
and a compound (vi) of formula X₅-S(O)-X₆, wherein X₅ and X₆, which can be the same or different, denote respectively a halogen atom.

M" preferably denotes Na or K, more preferably K.

X₅ and X₆, which can be the same or different, preferably denote Cl.

The initial molar ratio (compound of formula (i₆)/compound of formula (vi)) preferably ranges from 0.70 to 1.40, preferably from 0.90 to 1.30.

A solvent can be used as reaction medium to perform the reaction between compound of formula (i₆) with compound of formula (vi). An organic solvent, more preferably selected from non-polar and polar aprotic organic solvents, is more preferably used. Mention can be made of chlorinated solvents, such as dichloromethane, chloroform, o-dichlorobenzene, thionyl chloride, being preferably dichloromethane. The reaction can alternatively be performed without solvent. In this case, one or both of the reactants can serve as reaction medium.

In a sub-embodiment, compound (i₆) is first suspended in said solvent and then the obtained suspension is contacted with compound (vi) in order to perform the reaction step (1).

The reaction step between compound of formula (i₆) with compound of formula (vi) can be performed at a temperature ranging from 5°C to 80°C, preferably from 10°C to 70°C, more preferably from 15°C to 50°C. It can advantageously be performed at ambient temperature (20°C to 25°C). It is advantageous to perform this reaction step in two stages, by first contacting compound (i₆) with compound of formula (vi) at a temperature ranging from -5°C to 10°C, preferably from 0°C to 5°C, and preferably by maintaining the temperature below 10°C until completion of this contacting stage, and then by raising the temperature to a temperature ranging from 15°C to 50°C, preferably from 20°C to 25°C for the time necessary to complete the reaction.

The reaction between compound of formula (i₆) with compound of formula (vi) is preferably performed at atmospheric pressure.

Reaction time can vary widely as a function of the reaction temperature chosen. It can range from 1 hour to one day, especially from 1 hour to 12 hours, more particularly from 1 hour to 5 hours.

The progress of the reaction can be monitored by the degree of conversion of the compound of formula (i₆), which is the molar ratio of the amount of compound of formula (i₆) which has been consumed to the initial amount of compound of formula (i₆) in the reaction medium, this degree being readily calculated after dosing compound of formula (i₆) remaining in the reaction medium.

The reaction medium can then be treated in a way known *per se* in order to separate the different compounds present, especially to isolate the compound of formula (i₅) wherein n is equal to 1 obtained. It makes it possible for the remaining starting materials to be recycled in order to produce an additional amount of the targeted compound of formula (i₅) wherein n is equal to 1. One or more liquid/solid separation operations can be carried out, for example in order to separate possible solid impurities from the reaction medium. The techniques used can be crystallization, filtration on different types of supports, centrifugation, separation on settling and evaporation, this list not being exhaustive. Alternatively or in addition, one or more liquid/liquid separation operations can be carried out in order to separate and/or purify the product obtained. The techniques used can be distillation, liquid/liquid extraction, separation by reverse osmosis or separation by ion-exchange resins, this list not being exhaustive. These liquid/solid and liquid/liquid separation operations can be carried out under continuous or batch conditions, it being possible for a person skilled in the art to choose the most appropriate conditions.

Accordingly, the process for making compound of formula (i₅) wherein n is equal to 1 can additionally comprise a step which consists in isolating said compound (i₅), for instance by performing at least one liquid/solid separation and/or at least one liquid/liquid separation. In one specific embodiment, said liquid/solid separation is a filtration and said liquid/liquid separation is a distillation.

Preferably, isolated compound of formula formula (i₅) wherein n is equal to 1 has a purity degree of at least 94% wt, 95% wt, 96% wt, 97% wt, 98 % wt, or even 99 % wt.

The process for making compound of formula (i₅) wherein n is equal to 1 can additionally comprise a step which consists in separating part or all of the unreacted compound of formula (i₆), (vi) and in recycling these compounds in the process.

### Step (1bis)

The following conditions are identical to the ones described in connection with step (1): ratio of reactants, temperature, pressure, reaction time, monitoring of the degree of conversion, separation of the compounds present in the reaction medium after completion of the reaction, isolating of compound (VI), purity degree of compound (VI), recycling of the reactants. The other conditions are as follows.

The reaction can be performed in a solvent, advantageously a non polar solvent. Mention can be made of aromatic hydrocarbons such as benzene, toluene, xylenes, and ethylbenzene. Toluene is preferred. The reaction can alternatively be performed without solvent. In this case, compound (iv) can advantageously play the role of a solvent. It can therefore be added in slight excess relative to compound (i₅).

A catalyst can advantageously be used. It is preferably selected, from Lewis acids, such as, without limitation, titanium ethoxide (Ti(OEt)₄) or p-toluenesulfonic acid.

Before performing step (Ibis), it is possible to make the sulfone compound of formula (i₅) from the corresponding sulfoxide compound by performing an oxidation step by use of an oxidizing agent such as meta-chloroperoxybenzoic acid (mCPBA) .

### Step (1ter)

The following conditions are identical to the ones described in connection with step (1): ratio of reactants, solvent, temperature, pressure, reaction time, monitoring of the degree of conversion, separation of the compounds present in the reaction medium after completion of the reactions, isolating of compound (VI), purity degree of compound (VI), recycling of the reactants. The other conditions are as follows.

Compound (i5) is reacted with compound (iv') so as to form a compound of formula 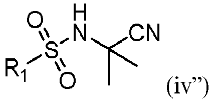 and then compound (iv") is reacted with a base.

Said base is preferably selected from trialkylamines, pyridines; and inorganic bases especially those selected from carbonates of alkali or alkaline earth metals, more preferably from carbonates of alkali metals, still more preferably from sodium or potassium carbonate. The amount of base to be added depends on the amount of compound (iv") formed. Typically, the molar ratio of base relative to compound (iv") ranges from 1:1 to 2:1, preferably from 1:1 to 1.6:1, more preferably from 1:1 to 1.3:1.

### Step (1quater)

Referring to compound of formula (i₅) used in step (1quater), among possible halogen atoms denoted by Y, chlorine is preferred.

Referring to said trialkylsilyl group in connection with the cyanide compound used in step (1quater), it is covalently bonded to the carbon atom of the cyanide function by its silicon atom. The alkyl chains in said trialkylsilyl group can be the same or different. They are preferably selected from C₁ to C₄ alkyl chains. Said trialkylsilyl group can be more specifically selected from trimethylsilyl, triethylsilyl, triisopropylsilyl, being preferably trimethylsilyl.

In one embodiment, the cyanide compound used in step (1quater) is in a salified form. In one sub-embodiment, the salt of cyanide compound can be represented by the formula M'^{q+}(C≡N⁻)_{q} wherein M'^{q+} is selected from metals and onium cations and q denotes the valency of said cation. Among suitable onium cations, mention can be made of ammonium, phosphonium, pyridinium, pyrazolinium, imidazolium, and arsenium cations. Ammonium cations can be especially selected from ammonium (NH₄⁺) and primary, secondary, tertiary, and quaternary ammonium cations, such as monoalkylammonium, dialkylammonium, trialkylammonium, tetraalkylammonium, trialkylbenzylammonium, and tetraarylammonium. Phosphonium cations can be especially selected from phosphonium (PH₄⁺) and quaternary phosphonium cations such as tetraalkylphosphonium, trialkylbenzylphosphonium, alkyltriarylphosphonium, and tetraarylphosphonium. In such quaternary ammonium and quaternary phosphonium cations, said alkyl, which can be the same or different, preferably denotes C₁-C₁₂ alkyl, more preferably C₄-C₆ alkyl; said aryl, which can be the same or different, preferably denotes phenyl or naphtalenyl.

According to an embodiment, the salt of cyanide compound possibly used in step (1quater) is an onium salt. In the sub-embodiment wherein the latter is represented by the formula M'^{q+}(C≡N⁻ )_{q} defined above, M'^{q+} is therefore selected from onium cations such as the ones described above. Preferably, M'^{q+} is a quaternary phosphonium or ammonium cation, more preferably a tetraalkylphosphonium or ammonium cation and even more preferably a tetrabutylphosphonium or ammonium cation. Phosphonium cations are preferred.

According to another embodiment, the salt of the cyanide compound possibly used in step (1quater) is a metal salt. In the sub-embodiment wherein the latter is represented by the formula M'^{q+}(⁻C≡N)_{q} defined above, M' is therefore at least one metal. Said metal or combination of metals can advantageously be selected from alkali metals, alkaline earth metals, and transition metals, more preferably from Li, Na, K, Mg, Ca, Fe, Co, Ag, Cu, even more preferably from K, Ag and Cu.

In one embodiment, the cyanide compound possibly used in step (1quater) is of formula M'CN such as M is selected from -Si(CH₃)₃ or is of formula M'^{q+}(CN⁻)_{q} wherein M'^{q+} is selected from alkali metal, alkaline earth metal, transition metal, tetraalkyl phosphonium, and tetraalkylammonium cations with q denoting the valency of said cation.

The initial molar ratio (compound of formula (i₅)/cyanide compound) preferably ranges from 0.70 to 1.40, preferably from 0.80 to 1.20.

A catalyst can optionally be used to perform the reaction. As suitable catalysts, mention can be made of Lewis bases and acids or any source of chloride, fluoride, bromide or cyanide. A bromide catalyst is particularly suitable when the cyanide compound M'CN is such as M' is a trialkylsilyl group.

A solvent is preferably used as reaction medium to perform the reaction between compound of formula (i₅) with the cyanide compound. A solvent that can solve, at least partly, the cyanide compound M'CN, especially selected from polar aprotic solvents, is preferable. An organic solvent is preferably used. Mention can be made of N,N-dimethylformamide, acetonitrile, dimethoxyethane, dichloromethane, being preferably dichloromethane. The reaction can alternatively be performed without solvent. In this case, one or both of the reactants can serve as reaction medium.

Temperature of the reaction step compound of formula (i₅) and the cyanide compound preferably ranges from 50°C to 150°C, preferably from 75°C to 125°C, more preferably from 90°C to 110°C, even more preferably from 95°C to 105°C.

The reaction is preferably performed at atmospheric pressure.

Reaction time can vary widely as a function of the reaction temperature chosen. It can range from 1 hour to one day, especially from 1 hour to 12 hours, more particularly from 1 hour to 5 hours.

The progress of the reaction can be monitored by the degree of conversion of the compound of formula (i₅), which is the molar ratio of the amount of compound of formula (i₅) which has been consumed to the initial amount of compound of formula (i₅) in the reaction medium, this degree being readily calculated after dosing compound of formula (i₅) remaining in the reaction medium.

The reaction medium can then be treated in a way known *per se* in order to separate the different compounds present, especially to isolate the compound of formula (VI) obtained. It makes it possible for the remaining starting materials to be recycled in order to produce an additional amount of the targeted compound of formula (VI). One or more liquid/solid separation operations can be carried out, for example in order to separate possible solid impurities from the reaction medium. The techniques used can be crystallization, filtration on different types of supports, centrifugation, separation on settling and evaporation, this list not being exhaustive. Alternatively or in addition, one or more liquid/liquid separation operations can be carried out in order to separate and/or purify the product obtained. The techniques used can be distillation, liquid/liquid extraction, separation by reverse osmosis or separation by ion-exchange resins, this list not being exhaustive. These liquid/solid and liquid/liquid separation operations can be carried out under continuous or batch conditions, it being possible for a person skilled in the art to choose the most appropriate conditions.

Accordingly, the process for making compound of formula (VI) can additionally comprise a step which consists in isolating said compound (VI), for instance by performing at least one liquid/solid separation and/or at least one liquid/liquid separation. In one specific embodiment, said liquid/solid separation is a filtration and said liquid/liquid separation is a distillation. For performing the filtration, temperature of the reaction medium is preferably cooled to a temperature ranging from 10 to 30 °C, preferably from 15°C to 25°C after the reaction step between compound of formula (i₅) and the cyanide compound.

The process can additionally comprise a step which consists in separating part or all of the unreacted compound of formula (i₅) and/or the cyanide compound and in recycling these compounds in the process.

### Step (1quinquies)

Referring to Y in compound (i₅) used in step (1quinquies), among possible -ORₘ groups wherein Rₘ is an alkali metal, Rₘ is preferably selected from K and Na, being more preferably K.

Referring to M' in the cyanide compound used in step (1quinquies), among possible halogen atoms, chlorine is preferred.

The initial molar ratio (compound of formula (i₅)/cyanide compound) preferably ranges from 0.30 to 0.70, preferably from 0.40 to 0.60.

A solvent is preferably used as reaction medium to perform the reaction between compound of formula (i₅) with the cyanide compound. Preferably it is an aqueous solvent. Water is particularly suitable.

According to one embodiment, prior to the reaction step between compound (i₅) and the cyanide compound, compound (i₅) is first dissolved in water. The solution obtained is preferably cooled to a temperature below 10°C, preferably below 5°C, more preferably below 0°C, and still more preferably below -1°C, at about -2°C. The cooled solution is then contacted with the cyanide compound.

For contacting of compound (i₅) (optionally in the form of the cooled aqueous solution described above) with the cyanide compound, it is preferable to maintain temperature from about - 5°C to about 5°C, preferably from about -2°C to about 1°C until completion of the contacting stage. Temperature can be maintained from about -5°C to about 5°C, preferably from about -2°C to about 1°C, and more preferably at about 0°C, for the time necessary to complete the reaction.

The reaction is preferably performed at atmospheric pressure.

Reaction time can vary widely as a function of the reaction temperature chosen. It can range from 1 hour to one day, especially from 1 hour to 12 hours, more particularly from 1 hour to 5 hours.

The progress of the reaction can be monitored by the degree of conversion of the compound of formula (i₅), which is the molar ratio of the amount of compound of formula (i₅) which has been consumed to the initial amount of compound of formula (i₅) in the reaction medium, this degree being readily calculated after dosing compound of formula (i₅) remaining in the reaction medium.

The reaction medium can then be treated in a way known *per se* in order to separate the different compounds present, especially to isolate the compound of formula (VI) obtained. It makes it possible for the remaining starting materials to be recycled in order to produce an additional amount of the targeted compound of formula (VI). One or more liquid/solid separation operations can be carried out, for example in order to separate possible solid impurities from the reaction medium. The techniques used can be crystallization, filtration on different types of supports, centrifugation, separation on settling and evaporation, this list not being exhaustive. Alternatively or in addition, one or more liquid/liquid separation operations can be carried out in order to separate and/or purify the product obtained. The techniques used can be distillation, liquid/liquid extraction, separation by reverse osmosis or separation by ion-exchange resins, this list not being exhaustive. These liquid/solid and liquid/liquid separation operations can be carried out under continuous or batch conditions, it being possible for a person skilled in the art to choose the most appropriate conditions.

Accordingly, the process for making compound of formula (VI) can additionally comprise a step which consists in isolating said compound (VI), for instance by performing at least one liquid/liquid separation. In one specific embodiment, the process comprises a liquid-liquid extraction and/or a distillation. Performing at least one liquid-liquid extraction is recommended if the reaction between compound (i5) and the cyanide compound is carried out in an aqueous reaction medium. For the liquid-liquid extraction, a (polar or non-polar) aprotic solvent is preferably used. Mention can be made of esters such as ethyl acetate, methyl acetate, ethyl propionate; chlorinated solvents such as dichloromethane; ethers such as diethyl ether; being preferably a chlorinated solvent and more preferably dichloromethane.

Preferably, isolated compound of formula (VI) has a purity degree of at least 94% wt, 95% wt, 96% wt, 97% wt, 98 % wt, or even 99 % wt. The process can additionally comprise a step which consists in separating part or all of the unreacted compound of formula (i₅) and/or the cyanide compound and in recycling these compounds in the process.

As examples of suitable compounds of formula (i₅), mention can be made of trifluoromethanesulfinic chloride, trifluoromethanesulfonyl chloride, trifluoromethanesulfinamide, trifluoromethanesulfonamide, potassium trifluoromethanesulfinate and potassium trifluoromethanesulfonate.

As examples of suitable compounds of formula (iv), mention can be made of propan-2-one oxime, propan-2-imine, propan-2-one.

As examples of suitable compounds of formula M'CN, mention can be made of cyanic chloride, or any of cyanosodium, cyanopotassium, hydrogen cyanide, trimethylsilyl cyanide, tetramethylammonium cyanide, tetraethylammonium cyanide, tetrapropylammonium cyanide, tetrabutylammonium cyanide, tetrapentylammonium cyanide, tetrahexylammonium cyanide, tetraheptylammonium cyanide, tetraoctylammonium cyanide, tetranonylammonium cyanide, tetradecylammonium cyanide, tetramethylphosphonium cyanide, tetraethylphosphonium cyanide, tetrapropylphosphonium cyanide, tetrabutylphosphonium cyanide, tetrapentylphosphonium cyanide, tetrahexylphosphonium cyanide, tetraheptylphosphonium cyanide, tetraoctylphosphonium cyanide, tetranonylphosphonium cyanide, tetradecylphosphonium cyanide.

Another subject-matter of the invention is the use as component for in an electrolyte composition, especially one suitable for electrochemical cells such as lithium ion batteries, of at least one compound of formula (VI) as described above.

Compound(s) of formula (VI) can advantageously be used for said electrolyte composition as solvent(s) or additive(s), depending on the amount added in the electrolyte composition.

One object of the present invention is accordingly an electrolyte composition, especially one suitable for an electrochemical cell such as a lithium-ion battery, comprising at least one compound selected from compounds (VI) described above or a mixture thereof and at least one electrolyte salt.

Said at least one compound of formula (VI) or mixtures thereof can advantageously be present in the electrolyte composition in an amount ranging from 0.05% to 95%, preferably from 0.8% to 70%, more preferably from 1% to 50%, more preferably from 2% to 20%, even more preferably from 3% to 10%, by weight relative to the total weight of the electrolyte composition.

According to an embodiment wherein said compound of formula (VI) is an additive, it may be present in the electrolyte composition in the range from 0.05 to about 20 percent by weight, based on the total weight of the electrolyte composition, for example in the range of from 0.05 to about 10 percent by weight, or from 0.1 to about 5.0 percent by weight, or from 0.3 to about 4.0 percent by weight, or from 0.5 to 2.0 percent by weight.

According to an embodiment wherein said compound of formula (VI) is a solvent, it may be present in the electrolyte composition in the range from about 20% to about 99,95% by weight of the electrolyte composition.

The electrolyte composition according to the invention can further comprise at least one of the following compounds:
- a compound of formula (VIII) 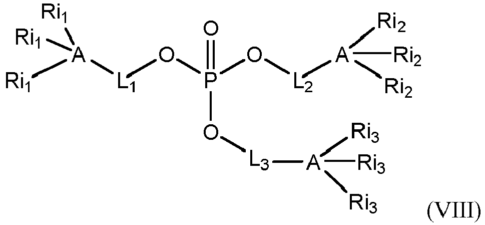
   wherein A is Si or C;
   L₁, L₂ and L₃, which can be the same or different, independently denote a substituted or unsubstituted, linear or branched, saturated or unsaturated, C₁-C₄ alkylene, C₃-C₁₂ cycloalkylene, C₃-C₁₂ arylene, or C₄-C₁₆ arylenealkylene group, optionally comprising at least one heteroatom selected from N, O and S;
   Ri₁, Ri₂ and Ri₃, which can be the same or different, independently denote H, a halogen atom, a linear or branched C₁-C₈ alkyl, C₂-C₈ alkenyl, C₂-C₈ alkynyl, or C₆-C₁₀ aryl group, optionally comprising at least one substituent selected from a halogens, hydroxyl, alkoxy, carbonyl, and carboxyl groups, and/or optionnaly comprising at least one heteroatom selected from N, O and S;
- a compound of formula (IX) 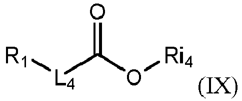
   wherein R₁ denotes H, a halogen atom, an alkyl group or a fluoroalkyl group;
   L₄ denotes a simple bond or a substituted or unsubstituted, linear or branched, saturated or unsaturated, C₁-C₄ alkylene, C₃-C₁₂ cycloalkylene, C₃-C₁₂ arylene, or C₄-C₁₆ arylenealkylene group, optionnaly comprising at least one heteroatom selected from N, O and S;
   Ri₄ denotes H, a linear or branched C₁-C₈ alkyl, C₂-C₈ alkenyl, C₂-C₈ alkynyl, or C₆-C₁₀ aryl group, optionally comprising at least one substituent selected from a halogens, hydroxyl, alkoxy, carbonyl, and carboxyl groups, and/or optionally comprising at least one heteroatom selected from N, O and S;
- a compound of formula (X) 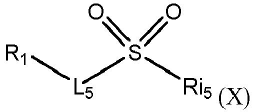
   wherein R₁ denotes H, a halogen atom, an alkyl group or a halogenoalkyl group;
   L₅ denotes a simple bond or a substituted or unsubstituted, linear or branched, saturated or unsaturated, C₁-C₄ alkylene, C₃-C₁₂ cycloalkylene, C₃-C₁₂ arylene, or C₄-C₁₆ arylenealkylene group, optionnaly comprising at least one heteroatom selected from N, O and S;
   Ri₅ denotes H, a linear or branched C₁-C₈ alkyl, C₂-C₈ alkenyl, C₂-C₈ alkynyl, or C₆-C₁₀ aryl group, optionally comprising at least one substituent selected from a halogens, hydroxyl, alkoxy, carbonyl, and carboxyl groups, and/or optionally comprising at least one heteroatom selected from N, O and S;
- a compound of formula (XI) 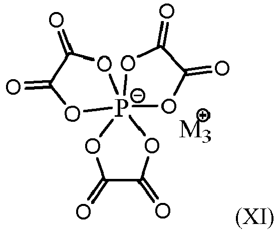 wherein M₃ is H, a metal or N(Ri₆Ri₇Ri₈Ri₉), wherein Ri₆, Ri₇, Ri₈ and Ri₉, which can be the same or different, independently denote H or a C₁-C₁₂ alkyl group.
- a compound of formula (XII) 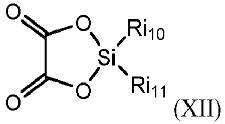 wherein Ri₁₀ and Ri₁₁, which can be the same or different, independently denote H, a C₁-C₈ alkyl, C₂-C₈ alkenyl, C₂-C₈ alkynyl, or C₆-C₁₀ aryl group, optionally comprising at least one substituent selected from halogens, hydroxyl, alkoxy, carbonyl, and carboxyl groups;
- a compound of formula (XIII) 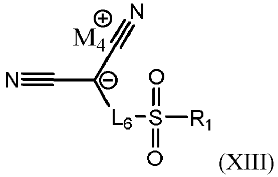
   wherein R₁ denotes H, a halogen atom, an alkyl group or a halogenoalkyl group;
   L₆ is a simple bond or a substituted or unsubstituted, linear or branched, saturated or unsaturated, C₁-C₄ alkylene, C₃-C₁₂ cycloalkylene, C₃-C₁₂ arylene, or C₄-C₁₆ arylenealkylene group, optionally comprising at least one heteroatom selected from N, O and S;
   M₄ is H, a metal or N(Ri₆Ri₇Ri₈Ri₉), wherein Ri₆, Ri₇, Ri₈ and Ri₉, which can be the same or different, independently denote H or a C₁-C₁₂ alkyl group;
- a compound of formula (XIV) 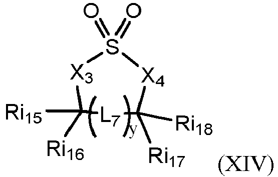 wherein X₃ and X₄, which can be the same or different, are independently selected from:
   ▪ NRi₁₂ where Ri₁₂ is H, a C₁-C₄ alkyl group, a C₁-C₄ alkenyl group or a C₁-C₄ alkynyl group;
   ▪ CRi₁₃Ri₁₄ where Ri₁₃ and Ri₁₄, which can be the same or different, are independently H, a C₁-C₄ alkyl group, a C₁-C₄ alkenyl group or a C₁-C₄ alkynyl group;
   ▪ and O;
      Ri₁₅, Ri₁₆, Ri₁₇ and Ri₁₈, which can be the same or different, are independently selected from H, a halogen atom, a C₁-C₄ alkyl group and a C₁-C₄ halogenoalkyl group;
      y is an integer ranging from 0 to 2 such as (L₇)₀ stands for a simple bond and when y is 1 or 2, L₇ stands for a CRi₁₉Ri₂₀ group where Ri₁₉ and Ri₂₀, which can be the same or different, are H, a C₁-C₄ alkyl group, a C₁-C₄ alkenyl group or a C₁-C₄ alkynyl group;
- a compound of formula (XV) 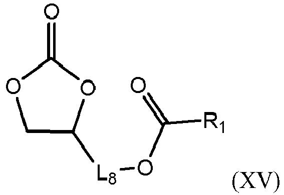
   wherein R₁ denotes H, a halogen atom, a C₁-C₄ alkyl group or a C₁-C₄ halogenoalkyl group;
   L8 is a simple bond or a substituted or unsubstituted, linear or branched, saturated or unsaturated, C₁-C₄ alkylene, C₃-C₁₂ cycloalkylene, C₃-C₁₂ arylene, or C₄-C₁₆ arylenealkylene group, optionnaly comprising at least one heteroatom selected from N, O and S;
- a compound of formula (XVI) 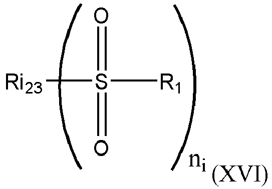
   wherein R₁ denotes H, a halogen atom, a C₁-C₄ alkyl group or a C₁-C₄ halogenoalkyl group; nᵢ denotes an integer greater than or equal to 2,
   Ri₂₃ denotes a nᵢ-valent linkage group comprising at least one carbon atom and atoms selected from C, H, a halogen atom and O, and the S atom of the -SO₂R₁ group is bound to a carbon atom of the Ri₂₃ group;
- a compound of formula (XVII) 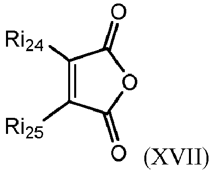 wherein Ri₂₄ and Ri₂₅, which can be the same or different, are independently selected from H, a halogen atom, a C₁-C₄ alkyl group, and a C₁-C₄ halogenoalkyl group;
- and mixtures thereof.

In connection with compound of formula (VIII), according to an embodiment, A is Si. In this case, L₁, L₂, and L₃ which can be the same or different, preferably independently denote a simple bond or a substituted or unsubstituted, linear or branched, saturated or unsaturated, C₁-C₄ alkylene, being more preferably a simple bond. Also according to this embodiment, Ri₁, Ri₂ and Ri₃, which can be the same or different, preferably independently denote a linear or branched C₁-C₈ alkyl, C₂-C₈ alkenyl, C₂-C₈ alkynyl, more preferably a linear or branched C₁-C₄ alkyl and still more preferably methyl.

Still in connection with compound of formula (VIII), according to another embodiment, A is C. In this case, L₁, L₂, and L₃ which can be the same or different, preferably independently denote a substituted or unsubstituted, linear or branched, saturated or unsaturated, C₁-C₄ alkylene, more preferably a C₁-C₃ alkylene, still more preferably a C₁-C₂ alkylene, even more preferably - CH₂-. Also according to this embodiment, Ri₁, Ri₂ and Ri₃, which can be the same or different, preferably independently denote a fluorine atom or a linear or branched C₁-C₄ fluoro or perfluoroalkyl group, being preferably F.

In connection with compound of formula (IX), L₄ denotes preferably a substituted or unsubstituted, linear or branched, saturated or unsaturated, C₁-C₄ alkylene, more preferably a C₁-C₃ alkylene, still more preferably a C₁-C₂ alkylene, even more preferably CH₂. Ri₄ preferably denotes a linear or branched C₁-C₈ alkyl, more preferably a C₁-C₄ alkyl, still more preferably a C₁-C₂ alkyl and even more preferably CH₃. R₁ preferably denotes a C₁-C₄ fluoro or perfluoroalkyl group, more preferably a C₁-C₃ fluoro or perfluoroalkyl group, still more preferably a C₁-C₂ fluoro or perfluoroalkyl group and even more preferably a C₁ fluoro or perfluoroalkyl group, such as in particular CF₃.

In connection with compound of formula (X), L₅ denotes preferably a simple bond or a substituted or unsubstituted, linear or branched, saturated or unsaturated, C₁-C₄ alkylene, more preferably a simple bond or a C₁-C₃ alkylene, still more preferably a simple bond or a C₁-C₂ alkylene, even more preferably a simple bond or CH₂. R₁ preferably denotes a C₁-C₄ fluoro or perfluoroalkyl group, more preferably a C₁-C₃ fluoro or perfluoroalkyl group, still more preferably a C₁-C₂ fluoro or perfluoroalkyl group and even more preferably a C₁ fluoro or perfluoroalkyl group. In one sub-embodiment, L₅ is a simple bond and R₁ is CH₂F or CF₃. In another sub-embodiment, L₅ is CH₂ and R₁ is CHF₂. In any cases, Ri₅ preferably denotes a linear or branched C₁-C₈ alkyl, more preferably a C₁-C₄ alkyl, still more preferably a C₁-C₂ alkyl and even more preferably CH₃.

In connection with compound of formula (XI), M₃ is preferably a metal selected from alkali metals and rare earth metals; more preferably from alkali metals, especially Li and Na; and still more preferably Li.

In connection with compound of formula (XII), Ri₁₀ and Ri₁₁, which can be the same or different, preferably independently denote a C₁-C₈ alkyl, more preferably a C₁-C₄ alkyl, still more preferably a C₁-C₂ alkyl and even more preferably CH₃.

In connection with compound of formula (XIII), R₁ preferably denotes a C₁-C₄ fluoro or perfluoroalkyl group, more preferably a C₁-C₈ fluoro or perfluoroalkyl group, still more preferably a C₁-C₂ fluoro or perfluoroalkyl group and even more preferably a C₁ fluoro or perfluoroalkyl group, such as in particular CF₃. L₆ preferably denotes a simple bond or a substituted or unsubstituted, linear or branched, saturated or unsaturated, C₁-C₄ alkylene, being more preferably a simple bond. M₄ is preferably a metal selected from alkali metals and rare earth metals; more preferably from alkali metals, especially Li and Na; and still more preferably Li.

In connection with compound of formula (XIV), according to one embodiment, X₃ is NRi₁₂ where Ri₁₂ is H, a C₁-C₄ alkyl group, a C₁-C₄ alkenyl group or a C₁-C₄ alkynyl group. Preferably Ri₁₂ is a C₁-C₄ alkyl group, more preferably a C₁-C₂ alkyl group and more preferably CH₃. In this case, X₄ is preferably CRi₁₃Ri₁₄ where Ri₁₃ and Ri₁₄, which can be the same or different, are independently H, a C₁-C₄ alkyl group, a C₁-C₄ alkenyl group or a C₁-C₄ alkynyl group. Ri₁₃ and Ri₁₄, are preferably independently H or a C₁-C₄ alkyl group, and more preferably both H. According to the same embodiment, Ri₁₅, Ri₁₆, Ri₁₇ and Ri₁₈, which can be the same or different, are preferably independently selected from H and a C₁-C₄ alkyl group, being more preferably H.

According to another embodiment, X₃ is O. In this case, X₄ is preferably O. According to the same embodiment, Ri₁₅, Ri₁₆, Ri₁₇ and Ri₁₈, which can be the same or different, are preferably independently selected from H and a halogen atom, more preferably from H and fluorine. In one sub-embodiment, Ri₁₅, Ri₁₆ and Ri₁₇ are both H and Ri₁₈ is fluorine.

Still in connection with compound of formula (XIV), y is preferably equal to 0 such as (L₇)₀ stands for a simple bond.

In connection with compound of formula (XV), R₁ preferably denotes a C₁-C₄ fluoro or perfluoroalkyl group, more preferably a C₁-C₃ fluoro or perfluoroalkyl group, still more preferably a C₁-C₂ fluoro or perfluoroalkyl group and even more preferably a C₁ fluoroalkyl group, such as in particular CHF₂. L₈ preferably denotes a substituted or unsubstituted, linear or branched, saturated or unsaturated, C₁-C₄ alkylene, being more preferably a C₁-C₃ alkylene, still more preferably a C₁-C₂ alkylene, even more preferably CH₂.

In connection with compound of formula (XVI), R₁ denotes preferably a halogen atom or a C₁-C₄ halogenoalkyl group, more preferably F or a C₁-C₄ fluoro or perfluoroalkyl group and still preferably R₁ is F. The index nᵢ preferably denotes an integer equal to 2 so that Ri₂₃ denotes a divalent linkage. Ri₂₃ preferably denotes an alkylene, more preferably a C₁-C₆ alkylene, a C₁-C₅ alkylene, a C₁-C₄ alkylene and more preferably a C₃ alkylene, especially C₃H₆.

In connection with compound of formula (XVII), Ri₂₄ and Ri₂₅, which can be the same or different, are preferably selected from H, F, a C₁-C₄ alkyl group, and a C₁-C₄ fluoroalkyl group. Ri₂₄ is preferably selected from F and a C₁-C₄ fluoroalkyl group, being preferably F. Ri₂₅ is preferably selected from H, F and a C₁-C₄ fluoroalkyl group, being more preferably selected from H and F. In one sub-embodiment, Ri₂₄ is F and Ri₂₅ is H. In another sub-embodiment, Ri₂₄ and Ri₂₅ are both F.

Said at least one compound of formula (VIII), (IX), (X), (XI), (XII), (XIII), (XIV), (XV), (XVI) or mixture thereof can be present in the electrolyte composition in an amount ranging from 0.05% to 94.5%, preferably from 0.8% to 70%, more preferably from 1% to 50%, more preferably from 2% to 20%, even more preferably from 3% to 10%, by weight relative to the total weight of the electrolyte composition.

The electrolyte composition according to the invention can further comprise at least one non-fluorinated cyclic carbonate, preferably selected from ethylene carbonate, propylene carbonate, vinylene carbonate, ethyl propyl vinylene carbonate, vinyl ethylene carbonate, dimethylvinylene carbonate, and mixtures thereof.

Said non-fluorinated cyclic carbonate can be present in the electrolyte composition in an amount ranging from 5% to 50%, preferably from 10% to 45%, more preferably from 12% to 40%, more preferably from 15% to 35%, even more preferably from 17% to 30%, by weight relative to the total weight of the electrolyte composition.

The electrolyte composition according to the invention can further comprise at least one non-fluorinated acyclic carbonate, preferably selected from ethyl methyl carbonate, dimethyl carbonate, diethyl carbonate, di-tert-butyl carbonate, dipropyl carbonate, di-tert-butyl carbonate, dipropyl carbonate, methyl propyl carbonate, methyl butyl carbonate, ethyl butyl carbonate, propyl butyl carbonate, dibutyl carbonate, and mixtures thereof.

Said non-fluorinated acyclic carbonate can be present in the electrolyte composition in an amount ranging from 5% to 50%, preferably from 10% to 45%, more preferably from 12% to 40%, more preferably from 15% to 35%, even more preferably from 17% to 30%, by weight relative to the total weight of the electrolyte composition.

The electrolyte composition according to the invention can further comprise at least one fluorinated carbonate, preferably selected from 4-fluoro-1,3-dioxolan-2-one; 4-fluoro-4-methyl-1,3-dioxolan-2-one; 4-fluoro-5-methyl-1,3-dioxolan-2-one; 4-fluoro-4,5-dimethyl-1,3-dioxolan-2-one; 4,5-difluoro-1,3-dioxolan-2-one; 4,5-difluoro-4-methyl-1,3-dioxolan-2-one; 4,5-difluoro-4,5-dimethyl-1,3-dioxolan-2-one; 4,4-difluoro-1,3-dioxolan-2-one; 4,4,5-trifluoro-1,3-dioxolan-2-one; 4,4,5,5-tetrafluoro-1,3-dioxolan-2-one; and mixtures thereof; being preferably 4-fluoro-1,3-dioxolan-2-one.

Said fluorinated carbonate can be present in the electrolyte composition in an amount ranging from 0.05% to 20%, preferably from 0.8% to 15%, more preferably from 1% to 10%, more preferably from 2% to 10%, even more preferably from 3% to 10%, by weight relative to the total weight of the electrolyte composition.

The electrolyte composition according to the invention can further comprise at least one compound selected from:
- a fluorinated acyclic carboxylic acid ester represented by the formula:

   R₁₃-COO-R₁₄,
- a fluorinated acyclic carbonate represented by the formula:

   R₁₅-OCOO-R₁₆,
- a fluorinated acyclic ether represented by the formula:

   R₁₇-O-R₁₈,
- and mixtures thereof,
wherein
i) R₁₃ is H, an alkyl group, or a fluoroalkyl group;
ii) R₁₅ and R₁₇ is each independently a fluoroalkyl group and can be either the same as or different from each other;
iii) R₁₄, R₁₆, and R₁₈ is each independently an alkyl group or a fluoroalkyl group and can be either the same as or different from each other;
iv) either or both of R₁₃ and R₁₄ comprises fluorine; and
v) R₁₃ and R₁₄, R₁₅ and R₁₆, R₁₇ and R₁₈, each taken as a pair, comprise at least two carbon atoms but not more than seven carbon atoms.
These compounds can advantageously be used as solvents in the electrolyte compositions according to the present invention (hereinafter referred to as the "fluorinated solvents").

Preferably, none of R₁₃, R₁₄, R₁₅, R₁₆, R₁₇, nor R₁₈ contains a FCH₂- group or a -FCH-group.

In another embodiment, R₁₃ and R₁₅ in the formula above do not contain fluorine, and R₁₄ and R₁₆ contain fluorine.

Suitable fluorinated acyclic carboxylic acid esters are represented by the formula:

R₁₃-COO-R₁₄

wherein
i) R₁₃ is H, an alkyl group, or a fluoroalkyl group;
ii) R₁₄ is an alkyl group or a fluoroalkyl group;
iii) either or both of R₁₃ and R₁₄ comprises fluorine; and
iv) R₁₃ and R₁₄, taken as a pair, comprise at least two carbon atoms but not more than seven carbon atoms.

In one embodiment, R₁₃ is H and R₁₄ is a fluoroalkyl group. In one embodiment, R₁₃ is an alkyl group and R₁₄ is a fluoroalkyl group. In one embodiment, R₁₃ is a fluoroalkyl group and R₁₄ is an alkyl group. In one embodiment, R₁₃ is a fluoroalkyl group and R₁₄ is a fluoroalkyl group, and R₁₃ and R₁₄ can be either the same as or different from each other. In one embodiment, R₁₃ comprises one carbon atom. In one embodiment, R₁₃ comprises two carbon atoms.

In another embodiment, R₁₃ and R₁₄ are as defined herein above, and R₁₃ and R₁₄, taken as a pair, comprise at least two carbon atoms but not more than seven carbon atoms and further comprise at least two fluorine atoms, with the proviso that neither R₁₃ nor R₁₄ contains a FCH₂-group or a -FCH- group.

In one embodiment, the number of carbon atoms in R₁₃ in the formula above is 1, 3, 4, or 5.

In another embodiment, the number of carbon atoms in R₁₃ in the formula above is 1.

Examples of suitable fluorinated acyclic carboxylic acid esters include without limitation CH₃-COO-CH₂CF₂H (2,2-difluoroethyl acetate, CAS No. 1550-44-3), CH₃-COO-CH₂CF₃ (2,2,2-trifluoroethyl acetate, CAS No. 406-95-1), CH₃CH₂-COO-CH₂CF₂H (2,2-difluoroethyl propionate, CAS No. 1133129-90-4), CH₃-COO-CH₂CH₂CF₂H (3,3-difluoropropyl acetate), CH₃CH₂-COO-CH₂CH₂CF₂H (3,3-difluoropropyl propionate), F₂CHCH₂-COO-CH₃, F₂CHCH₂-COO-CH₂CH₃, and F₂CHCH₂CH₂-COO-CH₂CH₃ (ethyl 4,4-difluorobutanoate, CAS No. 1240725-43-2), H-COO-CH₂CF₂H (difluoroethyl formate, CAS No. 1137875-58-1), H-COO-CH₂CF₃ (trifluoroethyl formate, CAS No. 32042-38-9), and mixtures thereof. According to a preferred embodiment, the fluorinated acyclic carboxylic acid ester comprises 2,2-difluoroethyl acetate (CH₃-COO-CH₂CF₂H). According to another preferred embodiment, the fluorinated acyclic carboxylic acid ester comprises 2,2-difluoroethyl propionate (CH₃CH₂-COO-CH₂CF₂H). According to another preferred embodiment, the fluorinated acyclic carboxylic acid ester comprises 2,2,2-trifluoroethyl acetate (CH₃-COO-CH₂CF₃). According to another preferred embodiment, the fluorinated acyclic carboxylic acid ester comprises 2,2-difluoroethyl formate (H-COO-CH₂CF₂H).

Suitable fluorinated acyclic carbonates are represented by the formula

R₁₅-OCOO-R₁₆

wherein
i) R₁₅ is a fluoroalkyl group;
ii) R₁₆ is an alkyl group or a fluoroalkyl group; and
iii) R₁₅ and R₁₆ taken as a pair comprise at least two carbon atoms but not more than seven carbon atoms.

In one embodiment, R₁₅ is a fluoroalkyl group and R₁₆ is an alkyl group. In one embodiment, R₁₅ is a fluoroalkyl group and R₁₆ is a fluoroalkyl group, and R₁₅ and R₁₆ can be either the same as or different from each other. In one embodiment, R₁₅ comprises one carbon atom. In one embodiment, R₁₅ comprises two carbon atoms.

In another embodiment, R₁₅ and R₁₆ are as defined herein above, and R₁₅ and R₁₆, taken as a pair, comprise at least two carbon atoms but not more than seven carbon atoms and further comprise at least two fluorine atoms, with the proviso that neither R₁₅ nor R₁₆ contains a FCH₂-group or a -FCH- group.

Examples of suitable fluorinated acyclic carbonates include without limitation CH₃-OC(O)O-CH₂CF₂H (methyl 2,2-difluoroethyl carbonate, CAS No. 916678-13-2), CH₃-OC(O)O-CH₂CF₃ (methyl 2,2,2-trifluoroethyl carbonate, CAS No. 156783-95-8), CH₃-OC(O)O-CH₂CF₂CF₂H (methyl 2,2,3,3-tetrafluoropropyl carbonate, CAS No.156783-98-1), HCF₂CH₂-OCOO-CH₂CH₃ (ethyl 2,2-difluoroethyl carbonate, CAS No. 916678-14-3), and CF₃CH₂-OCOO-CH₂CH₃ (ethyl 2,2,2-trifluoroethyl carbonate, CAS No. 156783-96-9).

Suitable fluorinated acyclic ethers are represented by the formula

R₁₇-O-R₁₈

wherein
i) R₁₇ is a fluoroalkyl group;
ii) R₁₈ is an alkyl group or a fluoroalkyl group; and
iii) R₁₇ and R₁₈ taken as a pair comprise at least two carbon atoms but not more than seven carbon atoms.

In one embodiment, R₁₇ is a fluoroalkyl group and R₁₈ is an alkyl group. In one embodiment, R₁₇ is a fluoroalkyl group and R₁₈ is a fluoroalkyl group, and R₁₇ and R₁₈ can be either the same as or different from each other. In one embodiment, R₁₇ comprises one carbon atom. In one embodiment, R₁₇ comprises two carbon atoms.

In another embodiment, R₁₇ and R₁₈ are as defined herein above, and R₁₇ and R₁₈, taken as a pair, comprise at least two carbon atoms but not more than seven carbon atoms and further comprise at least two fluorine atoms, with the proviso that neither R₁₇ nor R₁₈ contains a FCH₂-group or a -FCH- group.

Examples of suitable fluorinated acyclic ethers include without limitation HCF₂CF₂CH₂-O-CF₂CF₂H (CAS No. 16627-68-2) and HCF₂CH₂-O-CF₂CF₂H (CAS No. 50807-77-7).

As explained above, the electrolyte composition according to the invention may comprise, advantageously as solvent, a fluorinated acyclic carboxylic acid ester, a fluorinated acyclic carbonate, a fluorinated acyclic ether, or mixtures thereof. As used herein, the term "mixtures thereof" encompasses both mixtures within and mixtures between solvent classes, for example mixtures of two or more fluorinated acyclic carboxylic acid esters, and also mixtures of fluorinated acyclic carboxylic acid esters and fluorinated acyclic carbonates, for example. Non-limiting examples include a mixture of 2,2-difluoroethyl acetate and 2,2-difluoroethyl propionate; and a mixture of 2,2-difluoroethyl acetate and 2,2 difluoroethyl methyl carbonate.

The fluorinated acyclic carboxylic acid ester, the fluorinated acyclic carbonate and/or the fluorinated acyclic ether can be present in the electrolyte composition in an amount ranging from 5% to 95%, preferably from 10% to 80%, more preferably from 20% to 75%, more preferably from 30% to 70%, even more preferably from 50% to 70%, by weight relative to the total weight of the electrolyte composition.

The electrolyte composition according to the invention further comprises at least one electrolyte salt. Said electrolyte salt is preferably a lithium salt when the electrolyte composition is to be used in a lithium-ion battery. The lithium electrolyte salt is preferably selected from hexafluorophosphate (LiPF₆), lithium bis(trifluoromethyl)tetrafluorophosphate (LiPF₄(CF₃)₂), lithium bis(pentafluoroethyl)tetrafluorophosphate (LiPF₄(C₂F₅)₂), lithium tris(pentafluoroethyl)trifluorophosphate (LiPF₃(C₂F₅)₃), lithium bis(trifluoromethanesulfonyl)imide (LiN(CF₃SO₂)₂), lithium bis(perfluoroethanesulfonyl)imide LiN(C₂F₅SO₂)₂, LiN(C₂F₅SO₃)₂, lithium (fluorosulfonyl) (nonafluorobutanesulfonyl)imide, lithium bis(fluorosulfonyl)imide, lithium tetrafluoroborate, lithium perchlorate, lithium hexafluoroarsenate, lithium hexafluoroantimonate, lithium tetrachloroaluminate, LiAlO₄, lithium trifluoromethanesulfonate, lithium nonafluorobutanesulfonate, lithium tris(trifluoromethanesulfonyl)methide, lithium bis(oxalato)borate, lithium difluoro(oxalato)borate, Li₂B₁₂F₁₂₋ₓHₓ where x is an integer equal to 0 to 8, and mixtures of lithium fluoride and anion receptors such as B(OC₆F₅)₃. The lithium electrolyte salt is preferably selected from lithium hexafluorophosphate, lithium bis(fluorosulfonyl)imide and lithium bis(trifluoromethanesulfonyl)imide, and is preferably hexafluorophosphate.

The electrolyte salt is present in the electrolyte composition of the invention in an amount ranging from 5% to 20%, preferably from 6% to 18%, more preferably from 8% to 17%, more preferably from 9% to 16%, even more preferably from 11% to 16%, by weight relative to the total weight of the electrolyte composition.

One further object of the invention is the use of at least one compound of formula (VI) such as defined above, eventually in combination with at least one compound of formula (VIII) to (XVII) such as defined above, as component(s) of an electrolyte composition, especially one suitable for an electrochemical cells such as a lithium-ion battery.

One other object of the present invention is an electrochemical cell comprising:
(a) a housing;
(b) an anode and a cathode disposed in the housing and in ionically conductive contact with one another;
(c) an electrolyte composition according to the invention.

Especially, there is provided herein an electrochemical cell comprising a housing, an anode and a cathode disposed in the housing and in ionically conductive contact with one another, an electrolyte composition, as described herein above providing an ionically conductive pathway between the anode and the cathode, and a porous or microporous separator between the anode and the cathode. According to a preferred embodiment, the electrochemical cell is a lithium ion battery.

The housing may be any suitable container to house the electrochemical cell components. Housing materials are well-known in the art and can include, for example, metal and polymeric housings. While the shape of the housing is not particularly important, suitable housings can be fabricated in the shape of a small or large cylinder, a prismatic case, or a pouch. The anode and the cathode may be comprised of any suitable conducting material depending on the type of electrochemical cell. Suitable examples of anode materials include without limitation lithium metal, lithium metal alloys, lithium titanate, aluminum, platinum, palladium, graphite, transition metal oxides, and lithiated tin oxide. Suitable examples of cathode materials include without limitation graphite, aluminum, platinum, palladium, electroactive transition metal oxides comprising lithium or sodium, indium tin oxide, and conducting polymers such as polypyrrole and polyvinylferrocene.

The porous separator serves to prevent short circuiting between the anode and the cathode. The porous separator typically consists of a single-ply or multi-ply sheet of a microporous polymer such as polyethylene, polypropylene, polyamide, polyimide or a combination thereof. The pore size of the porous separator is sufficiently large to permit transport of ions to provide ionically conductive contact between the anode and the cathode, but small enough to prevent contact of the anode and cathode either directly or from particle penetration or dendrites which can form on the anode and cathode. Examples of porous separators suitable for use herein are disclosed in U.S. Application SN 12/963,927 (filed 09 Dec 2010, U.S. Patent Application Publication No. 2012/0149852, now U.S. Patent No. 8,518,525).

Many different types of materials are known that can function as the anode or the cathode. In some embodiments, the cathode can include, for example, cathode electroactive materials comprising lithium and transition metals, such as LiCoO₂, LiNiO₂, LiMn₂O₄, LiCo_{0.2}Ni_{0.2}O₂, LiV₃O₈, LiNi_{0.5}Mn_{1.5}O₄; LiFePO₄, LiMnPO₄, LiCoPO₄, and LiVPO₄F. In other embodiments, the cathode active materials can include, for example:
- LiₐCoG_{b}O₂, where 0.90 ≤ a ≤ 1.8, and 0.001 ≤ b ≤ 0.1;
- LiₐNi_{b}Mn_{c}Co_{d}RₑO_{2-f}Z_{f}, where 0.8 ≤ a ≤ 1.2, 0.1 ≤ b ≤ 0.9, 0.0 ≤ c ≤ 0.7, 0.05 ≤ d ≤ 0.4, 0 ≤ e ≤ 0.2, wherein the sum of b+c+d+e is about 1, and 0≤f≤0.08;
- LiₐA_{1-b},R_{b}D₂, where 0.90 ≤ a ≤ 1.8 and 0 ≤ b ≤ 0.5;
- LiₐE_{1-b}R_{b}O_{2-c}D_{c}, where 0.90 ≤ a ≤ 1.8, 0 ≤ b ≤ 0.5 and 0 ≤ c ≤ 0.05;
- LiₐNi_{1-b-c}CO_{b}R_{c}O_{2-d}Z_{d}, where 0.9 ≤ a ≤ 1.8, 0 ≤ b ≤ 0.4, 0 ≤ c ≤ 0.05, and 0 ≤ d ≤ 0.05;
- Li_{1+z}Ni_{1-x-y}CoₓAl_{y}O₂, where 0 < x < 0.3, 0 < y <0.1, and 0 < z < 0.06.

In the above chemical formulas, A is Ni, Co, Mn, or a combination thereof; D is O, F, S, P, or a combination thereof; E is Co, Mn, or a combination thereof; G is Al, Cr, Mn, Fe, Mg, La, Ce, Sr, V, or a combination thereof; R is Al, Ni, Co, Mn, Cr, Fe, Mg, Sr, V, Zr, Ti, a rare earth element, or a combination thereof; Z is F, S, P, or a combination thereof. Suitable cathodes include those disclosed in U.S. Patent Nos. 5,962,166; 6,680,145; 6,964,828; 7,026,070; 7,078,128; 7,303,840; 7,381,496; 7,468,223; 7,541,114; 7,718,319; 7,981,544; 8,389,160; 8,394,534; and 8,535,832, and the references therein. By "rare earth element" is meant the lanthanide elements from La to Lu, and Y and Sc.

In another embodiment the cathode material is an NMC cathode; that is, a LiNiMnCoO cathode, more specifically, cathodes in which
- the atomic ratio of Ni:Mn:Co is 1:1:1 (LiₐNi_{1-b-c}CO_{b}R_{c}O_{2-d}Z_{d} where 0.98 ≤ a ≤ 1.05, 0 ≤ d ≤ 0.05, b = 0.333, c = 0.333, where R comprises Mn); or
- the atomic ratio of Ni:Mn:Co is 5:3:2 (LiₐNi_{1-b-c}CO_{b}R_{c}O_{2-d}Z_{d} where 0.98 ≤ a ≤ 1.05, 0 ≤ d ≤ 0.05, c = 0.3, b = 0.2, where R comprises Mn).

In another embodiment, the cathode comprises a material of the formula LiₐMn_{b}J_{c}O₄Z_{d}, wherein J is Ni, Co, Mn, Cr, Fe, Cu, V, Ti, Zr, Mo, B, Al, Ga, Si, Li, Mg, Ca, Sr, Zn, Sn, a rare earth element, or a combination thereof; Z is F, S, P, or a combination thereof; and 0.9 ≤ a ≤ 1.2, 1.3 ≤ b ≤ 2.2, 0 ≤ c ≤ 0.7, 0 ≤ d ≤ 0.4.

In another embodiment, the cathode in the electrochemical cell or lithium ion battery disclosed herein comprises a cathode active material exhibiting greater than 30 mAh/g capacity in the potential range greater than 4.6 V versus a Li/Li⁺ reference electrode. One example of such a cathode is a stabilized manganese cathode comprising a lithium-containing manganese composite oxide having a spinel structure as cathode active material. The lithium-containing manganese composite oxide in a cathode suitable for use herein comprises oxides of the formula LiₓNi_{y}M_{z}Mn_{2-y-z}O_{4-d}, wherein x is 0.03 to 1.0; x changes in accordance with release and uptake of lithium ions and electrons during charge and discharge; y is 0.3 to 0.6; M comprises one or more of Cr, Fe, Co, Li, Al, Ga, Nb, Mo, Ti, Zr, Mg, Zn, V, and Cu; z is 0.01 to 0.18; and d is 0 to 0.3. In one embodiment in the above formula, y is 0.38 to 0.48, z is 0.03 to 0.12, and d is 0 to 0.1. In one embodiment in the above formula, M is one or more of Li, Cr, Fe, Co and Ga. Stabilized manganese cathodes may also comprise spinel-layered composites which contain a manganese-containing spinel component and a lithium rich layered structure, as described in U.S. Patent No. 7,303,840.

In another embodiment, the cathode comprises a composite material represented by the structure of Formula:

x(Li_{2-w}A₁₋ᵥQ_{w+v}O₃₋ₑ)•(1-x)(Li_{y}Mn_{2-z}M_{z}O_{4-d})

wherein:
- x is about 0.005 to about 0.1;
- A comprises one or more of Mn or Ti;
- Q comprises one or more of Al, Ca, Co, Cr, Cu, Fe, Ga, Mg, Nb, Ni, Ti, V, Zn, Zr or Y;
- e is 0 to about 0.3;
- v is 0 to about 0.5.
- w is 0 to about 0.6;
- M comprises one or more of Al, Ca, Co, Cr, Cu, Fe, Ga, Li, Mg, Mn, Nb, Ni, Si, Ti, V, Zn, Zr or Y;
- d is 0 to about 0.5;
- y is about 0 to about 1; and
- z is about 0.3 to about 1; and
wherein the Li_{y}Mn_{2-z}M_{z}O_{4-d} component has a spinel structure and the Li_{2-w}Q_{w+v}A₁₋ᵥO₃₋ₑ component has a layered structure.

In the above formula, x can be preferably about 0 to about 0.1.

In another embodiment, the cathode in the lithium ion battery disclosed herein comprises

LiₐA₁₋ₓRₓDO_{4-f}Z_{f},

wherein:
- A is Fe, Mn, Ni, Co, V, or a combination thereof;
- R is Al, Ni, Co, Mn, Cr, Fe, Mg, Sr, V, Zr, Ti, a rare earth element, or a combination thereof;
- D is P, S, Si, or a combination thereof;
- Z is F, Cl, S, or a combination thereof;
- 0.8 ≤ a ≤ 2.2;
- 0 ≤x≤0.3; and
- 0≤f≤0.1.

In another embodiment, the cathode in the lithium ion battery ore electrochemical cell disclosed herein comprises a cathode active material which is charged to a potential greater than or equal to about 4.1 V, or greater than or equal to 4.35 V, or greater than 4.5 V, or greater than or equal to 4.6 V versus a Li/Li⁺ reference electrode. Other examples are layered-layered high-capacity oxygen-release cathodes such as those described in U.S. Patent No. 7,468,223 charged to upper charging potentials above 4.5 V.

In some embodiments, the cathode comprises a cathode active material exhibiting greater than 30 mAh/g capacity in the potential range greater than 4.6 V versus a Li/Li⁺ reference electrode, or a cathode active material which is charged to a potential greater than or equal to 4.35 V versus a Li/Li⁺ reference electrode.

A cathode active material suitable for use herein can be prepared using methods such as the hydroxide precursor method described by Liu et al (J. Phys. Chem. C 13:15073-15079, 2009). In that method, hydroxide precursors are precipitated from a solution containing the required amounts of manganese, nickel and other desired metal(s) acetates by the addition of KOH. The resulting precipitate is oven-dried and then fired with the required amount of LiOH•H₂0 at about 800 to about 1000°C in oxygen for 3 to 24 hours. Alternatively, the cathode active material can be prepared using a solid phase reaction process or a sol-gel process as described in U.S. Patent No. 5,738,957 (Amine).

A cathode, in which the cathode active material is contained, suitable for use herein may be prepared by methods such as mixing an effective amount of the cathode active material (e.g. about 70 wt% to about 97 wt%), a polymer binder, such as polyvinylidene difluoride, and conductive carbon in a suitable solvent, such as N-methylpyrrolidone, to generate a paste, which is then coated onto a current collector such as aluminum foil, and dried to form the cathode.

An electrochemical cell or lithium ion battery as disclosed herein further contains an anode, which comprises an anode active material that is capable of storing and releasing lithium ions. Examples of suitable anode active materials include, for example, lithium alloys such as lithium-aluminum alloy, lithium-lead alloy, lithium-silicon alloy, and lithium-tin alloy; carbon materials such as graphite and mesocarbon microbeads (MCMB); phosphorus-containing materials such as black phosphorus, MnP₄ and CoP₃; metal oxides such as SnO₂, SnO and TiO₂; nanocomposites containing antimony or tin, for example nanocomposites containing antimony, oxides of aluminum, titanium, or molybdenum, and carbon, such as those described by Yoon et al (Chem. Mater. 21, 3898-3904, 2009); and lithium titanates such as Li₄Ti₅O₁₂ and LiTi₂O₄. In one embodiment, the anode active material is lithium titanate or graphite. In another embodiment, the anode is graphite.

An anode can be made by a method similar to that described above for a cathode wherein, for example, a binder such as a vinyl fluoride-based copolymer is dissolved or dispersed in an organic solvent or water, which is then mixed with the active, conductive material to obtain a paste. The paste is coated onto a metal foil, preferably aluminum or copper foil, to be used as the current collector. The paste is dried, preferably with heat, so that the active mass is bonded to the current collector. Suitable anode active materials and anodes are available commercially from companies such as Hitachi, NEI Inc. (Somerset, NJ), and Farasis Energy Inc. (Hayward, CA).

The electrochemical cell as disclosed herein can be used in a variety of applications. For example, the electrochemical cell can be used for grid storage or as a power source in various electronically powered or assisted devices ("Electronic Device") such as a computer, a camera, a radio, a power tool, a telecommunications device, or a transportation device (including a motor vehicle, automobile, truck, bus or airplane).

One other object of the present invention is an electronic device, a transportation device, or a telecommunications device, comprising an electrochemical cell according to the invention.

One other object of the present invention is a method for forming an electrolyte composition. The method comprises combining a) at least one compound selected from those of formula (VI), b) at least one electrolyte salt, c) optionally at least one compound selected from those of formula (VIII) to (XVII), to form the electrolyte composition. In some embodiments, other components, especially such as those described above in connection with the electrolyte composition of the invention, are combined according to this method. Mention can be made of non-fluorinated cyclic carbonates, non-fluorinated acyclic carbonates, fluorinated cyclic carbonates, fluorinated acyclic carboxylic acids, fluorinated acyclic carbonates and/or fluorinated acyclic ethers described above. The components can be combined in any suitable order. The step of combining can be accomplished by adding the individual components of the electrolyte composition sequentially or at the same time. In some embodiments, the components a) and c) are combined to make a first solution. After the formation of the first solution, an amount of the electrolyte salt is added to the first solution in order to produce the electrolyte composition having the desired concentration of electrolyte salt. Alternatively, the components a) and b) are combined to make a first solution, and after the formation of the first solution an amount of component c) and/or the other optional components is added to produce the electrolyte composition. Typically, the electrolyte composition is stirred during and/or after the addition of the components in order to form a homogeneous mixture.

Should the disclosure of any patents, patent applications, and publications which are incorporated herein by reference conflict with the description of the present application to the extent that it may render a term unclear, the present description shall take precedence.

The claims are integral part of the description of the present application.

The invention will now be further described in examples without intending to limit it.

### EXAMPLES

The present invention is further defined in the following examples. It should be understood that these examples, while indicating preferred embodiments of the invention, are given by way of illustration only. From the above discussion and these examples, one skilled in the art can ascertain the essential characteristics of this invention, and without departing from the spirit and scope thereof, can make various changes and modifications of the invention to adapt it to various uses and conditions.

### Preparation of the compounds

Otherwise indicated, the raw materials used are commercially available. Mention can be made especially of: o-dichlorobenzene (Sigma-Aldrich, Germany), dichloromethane (DCM) (Sigma-Aldrich, Germany), xylene (Sigma-Aldrich, Germany), trimethylamine (Sigma-Aldrich, Germany), acetone oxime (Sigma-Aldrich, Germany), potassium cyanide (Sigma-Aldrich, Germany), silver cyanide (Sigma-Aldrich, Germany), thionyl chloride (Sigma-Aldrich, Germany), cyanogen chloride (prepared according to Journal of the American Chemical Society, 1919, vol. 41, p. 1241-1248), adiponitrile (Sigma-Aldrich, Germany), acetonitrile (Sigma-Aldrich, Germany).

### Example 1.1: synthesis of N-trifluoromethylsulfonylpropaneimine (compound (VI).1)

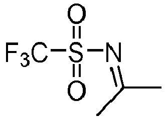

To a solution of triflyl chloride CF₃SO₂Cl (168.5g, 1 mol) and triethylamine (102g, 1mol) in methylene chloride (DCM, 500ml) maintained at 0-5°C is added a solution of 2-propaneimine* (50g, 0.88 mol) in DCM (200ml); the temperature is maintained at 5-10°C during the addition and then raised to 20°C; the corresponding suspension is stirred during 1h.
After filtration of the crude mixture, N-trifluoromethylsulfonylpropaneimine is isolated by vacuum distillation (115g, yield 60%, purity > 95% by GC).
* 2-propaneimine is prepared according to the literature: Bock, Hans; Dammel, Ralph - Chemische Berichte, 1987, vol. 120, p. 1961 - 1970

### Example 1.2: synthesis of N-trifluoromethylsulfonylpropaneimine (compound (VI).1)

Potassium triflinate CF₃SO₂K (200g, 1.16 mol) obtained by reduction of triflyl chloride according to a known method described by R.M. Scribner, J. Org. Chem., 31, 3671 (1966), is suspended in DCM (500ml) maintained at 0-5°C; thionyl chloride SOCl₂ (119g, 1mol) is added to this suspension maintained at 0-5°C; the temperature is then raised to 20°C and the corresponding suspension is stirred during 1h; After filtration of the crude mixture and evaporation of the solvent, triflinyl chloride is isolated by distillation (120g, yield 68%, purity > 95% by quantitative ¹⁹F NMR).
To a solution of acetone oxime (44g, 0,6 mol) and triethylamine (61g, 0,6mol) in methylene chloride (DCM, 300ml) maintained at 0-5°C is added triflinyl chloride ((80g, 0,52 mol) in DCM (300ml); the temperature is maintained at 5-10°C during the addition and then raised to 20°C; the corresponding suspension is stirred during 1h.
After filtration of the crude mixture and evaporation of the solvent, N-trifluoromethylsulfonylpropaneimine is isolated by vacuum distillation (75g, yield 67%, purity > 95% by quantitative ¹⁹F NMR).

### Example 1.3: synthesis of Triflyl cyanide (compound (VI).9)

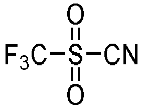

Triflyl chloride CF₃SO₂Cl (169g, 1mol) is dissolved *o*-dichlorobenzene (ODCB, 0.5L) containing potassium cyanide (80g, 1.2 equivalents) ; the resulting suspension is heated to 100°C during 5h, then cooled to 20°C and filtered.
Distillation under vacuum of the solution thus obtained leads to the isolation of triflyl cyanide as a colorless liquid (127g, yield 80%, purity > 95% by ¹⁹F NMR).

### Example 1.4: synthesis of Triflyl cyanide (compound (VI).9)

The synthesis was performed as in example 6.3 except that acetonitrile was used instead of o-dichlorobenzene.

### Example 1.5: synthesis of Triflyl cyanide (compound (VI).9)

The synthesis was performed as in example 6.3 except that adiponitrile was used instead of o-dichlorobenzene.

### Example 1.6: synthesis of Triflyl cyanide (compound (VI).9)

The synthesis was performed as in example 6.3 except that xylene was used instead of o-dichlorobenzene.

### Example 1.7: synthesis of Triflyl cyanide (compound (VI).9)

The synthesis was performed as in example 6.3 except that silver cyanide was used instead of potassium cyanide.

### Example 1.7: synthesis of Triflyl cyanide (compound (VI).9)

The synthesis was performed as in example 6.3 except that copper cyanide was used instead of potassium cyanide.

### Example 1.8: synthesis of Triflyl cyanide (compound (VI).9)

Pure potassium triflinate CF₃SO₂K (172g, 1 mol), obtained by reduction of triflyl chloride according to a known method described by R.M. Scribner, J. Org. Chem., 31, 3671 (1966), is dissolved in water (0.5L) and the resulting solution is cooled to -2 °C. Gaseous cyanogen chloride (123g, 2 mol) was passed into the solution over 25 min at -2 to +1 °C, and the cloudy mixture was stirred for 45 min at 0 °C before addition of CH₂Cl₂ (200 mL). The phases were separated, and the aqueous phase was extracted with CH₂Cl₂ (2 X 150 mL). Evaporation of the solvent from the combined organic layers and distillation of the residue gave CF₃SO₂CN (119 g, 75%).

### Preparation of the electrolyte compositions

In addition to the compounds synthesized in examples 1.1 to 1.8, the following battery grades compounds (called hereinafter compounds C) are used for the preparation of the electrolytes composition:
- tris(trimethylsilyl) phosphate (CAS 10497-05-09) (Sigma-Aldrich, Germany),
- tris(trifluoroethyl)phosphate (CAS 358-63-4) (Tokyo Chemical Industry, Japan),
- methyl 3,3,3-trifluoropropanonate (CAS 18830-44-9) (TCI Chemicals, India),
- 1,1-difluoro-2-(methylsulfonyl)ethane (CAS 1214268-07-1), prepared by the method described in WO 2015/051141
- (fluoromethylsulfonyl)methane (CAS 94404-44-1), prepared from dimethylsulfoxide and (diethylamino)sulfur trifluoride according to the method described in J-R. McCarthy, J. Am. Soc., 107, 735-736 (1985),
- trifluoro(methylsulfonyl)methane (CAS 421-82-9) (Apolloscientific, UK),
- lithium biscyano(trifluoromethylsulfonyl)methide (CAS 210043-24-6), prepared according to example 2 of US6576159 from malononitrile, lithium hydride and 1-(trifluoromethanesulfonyl)imidazole,
- lithium tris (oxalato)phosphate (CAS 321201-33-6), prepared according to examples 1 and 2 of EP1203001,
- 2,2-dimethyl-1,3,2-dioxasilolane-4,5-dione, prepared according to example "synthesis of dimethylsilyl oxalate" of WO2018/033357 from Solvay,
- 2-methylisothiazolidine-1,1-dioxide (CAS 83634-83-7) (Parchem, USA)
- 4-fluoro-1,
- 3,2-dioxathiolane-2,2-dioxide (CAS 23910-98-7) prepared according to example 2 of CN105541789,
- (2-oxo-1,3-dioxolan-4-yl)-2,2-difluoromethylacetate (CAS 1337958-06-1), prepared according to example 2 of US8735005,
- 1,3-Propanedisulfonyl difluoride (CAS 110073-91-1) prepared according to Journal of Fluorine Chemistry, 1997, vol. 83, # 2, p. 145-149,
- 3-Fluoro-2,5-furandione (CAS 2714-23-0) (Angene, UK)
- 3,4-Diffluoro-2,5-furandione (CAS 669-78-3) (Carbosynth)

Ethylene carbonate (EC), ethyl methyl carbonate (EMC), and dimethyl carbonate (DMC), all battery grade, were purchased at ENchem. Lithium hexafluorophosphate (LiPF₆), battery grade, was purchased at ENchem. 4-fluoro-1,3-dioxolane-2-one (FEC), battery grade, was purchased at Solvay. 2,2-difluoroethyl acetate (DFEA) was purchased at Solvay. 4,5-difluoro-1,3-dioxolane-2-one, battery grade, was provided by Solvay. It was obtained by a fluorination process of ethylene carbonate such as described in EP 2483231.

Electrolyte compositions are prepared by first mixing the solvents EC, EMC, DMC in their respective volume ratios EC/EMC/DMC (2:2:6) and dissolving the lithium salt LiPF₆ in the appropriate amount to yield 1.5 M composition. Compounds obtained from examples 1.1 to 1.8 are respectively added to each electrolyte composition in an amount of 2% (For each electrolyte composition prepared, the amount of added compound is given in weight relative to the total weight of the composition). Other electrolyte compositions are prepared in the same manner but by adding further at least one of the compounds C in an amount of 2% in weight relative to the total weight of the composition. As the results of moisture measurement, the moisture content of all the electrolytes compositions is under 10 mg/kg of composition (Karl Fisher method).

### Preparation of the pouch cells

Pouch cells are purchased from Pred Materials (New York, N.Y.) and are 600 mAh cells containing an NMC 532 cathode and a graphitic anode.
Before use, the pouch cells are dried in the antechamber of a dry box under vacuum 4 days at 55°C and vacuum -100kPa. Approximately 2.0 gram of electrolyte composition is injected through the bottom, and the bottom edge sealed in a vacuum sealer. For each example, two pouch cells are prepared using the same electrolyte composition.

### Pouch cells Assembly and Formation

The cells are held in an environmental chamber (model BTU-433, Espec North America, Hudsonville, Michigan) and evaluated using a battery tester (Series 4000, Maccor, Tulsa, OK) for the formation procedures (at 25 °C, 60 °C) and the high temperature cycling (at 45 °C).
The pouch cells are conditioned using the following cycling procedure. In a first cycle, the cell is charged for 3 hours at 0.1 C, corresponding to approximately 30 % state of charge; this is followed by 24 hour rest at 60 °C. The pouch cell is degassed and resealed in a vacuum sealer. The cell is pressed using hot press at 70 °C during 3 sec.
For the second cycle, the cell is charged at constant current (CC charge) of 0.5 C to 4.35 V followed by a CV voltage-hold step at 4.35 V until current dropped below 0.05C and rested 10min. This is followed by a CC discharge at 0.5C to 3.0 V and rested 10min. This cycle is repeated 3 times and it is used as a check of the capacity of the cell.
The final step for formation of pouch cell is charged at constant current (CC charge) of 0.5C to SOC30.
For the 25 °C cycles and the 45 °C cycling described below, the cells also have a 10 min rest following each charge and each discharge step.

### Cycling method

The cells are placed in an environmental chamber at 25°C and 45 °C and cycled: CC charge 1C to 4.35 V and CV charge to 0.05C, and CC discharge at 1C to 3.0 V.

### Storage procedure

The cells are placed in an environmental chamber at 70 °C with SOC 100, CC charge 1C to 4.35 V and CV charge to 0.05C, initial thickness checked. After 1 week later, they are put out from oven, the thickness is measured by Vernier calipers, the residual and recovery capacity is measured with CC discharge at 1C to 3.0V, and DC-IR is checked.

### Ionic conductivity measure procedure

The electrolytes are measured by LCR meter in temperature control chamber at -20°C even 60°C.

### Results

The tests show that the cells containing the electrolyte compositions according to the invention comprising at least one compound selected from those of formulae (VI) optionally in combination with at least one compound selected from those of formulae (VIII) to (XVII) have improved performance characteristics, especially regarding quality of the solid electrolyte interphase (SEI) formed on the electrodes surface, chemical stability, ionic conductivity, thermal stability, reversible capacity, cycle characteristics and/or gas generation.

## Claims

1. Compound of formula (VI) 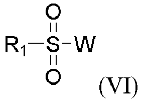 wherein R₁ denotes H, a halogen atom, an alkyl group or an halogenoalkyl group; and W denotes a group selected from groups (A), (B) and (C) 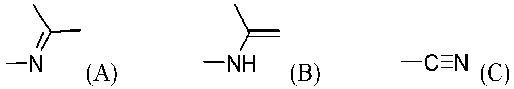 provided that R₁ is different from -CH₃ when W is of formula (C).

2. Compound according to claim 1 wherein R₁ denotes H, F, an alkyl group or a fluoroalkyl group; preferably F, an alkyl group or a fluoroalkyl group; more preferably F or a fluoroalkyl group; still more preferably a C₁-C₄ fluoro or perfluoroalkyl group and even more preferably CF₃.

3. Process for making a compound of formula (VI) 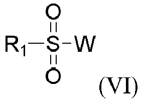 wherein R₁ denotes H, a halogen atom, an alkyl group or a halogenoalkyl group; and W denotes a group selected from groups (A), (B) and (C) 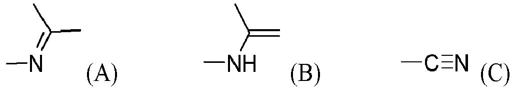 which comprises:
- a step (1) of reacting a compound of formula (i5) 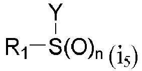 wherein R₁ denotes H, a halogen atom, an alkyl group or a halogenoalkyl group; Y denotes a halogen atom different from R₁ ; and n is an integer equal to 1 or 2;
with a compound of formula (iv) 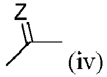 wherein Z denotes NH or NOH,
- or a step (Ibis) of reacting a compound of formula (i5) 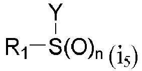 wherein R₁ denotes H, a halogen atom, an alkyl group or a halogenoalkyl group; Y denotes a-NH₂ group; and n is an integer equal to 2;
with a compound of formula (iv) 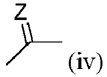 wherein Z denotes O,
- or a step (Iter) of reacting a compound of formula (i5) 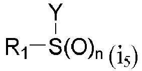 wherein R₁ denotes H, a halogen atom, an alkyl group or a halogenoalkyl group; Y denotes a halogen atom different from R₁; and n is an integer equal to 2;
with a compound of formula (iv') 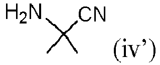 so as to form a compound of formula 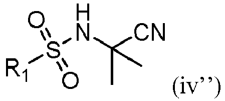 and then react compound (iv") with a base
- or a step (1quater) of reacting a compound of formula (i5) 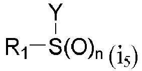
wherein R₁ denotes H, a halogen atom, an alkyl group or a halogenoalkyl group; Y denotes a halogen atom different from R₁ ; and n is an integer equal to 2;
with a cyanide compound of formula M'C=N wherein M' is selected from H and a trialkylsilyl group or a salt thereof,
- or a step (1quinquies) of reacting a compound of formula (i5) 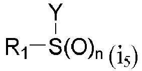
wherein R₁ denotes H, a halogen atom, an alkyl group or a halogenoalkyl group; Y denotes a-ORₘ group wherein Rₘ is an alkali metal or an alkaline earth metal; and n is an integer equal to 1;
with a cyanide compound of formula M'C=N wherein M' is a halogen atom,
provided that R₁ is different from -CH₃ when Z is NOH, Y is Cl and n is equal to 1 or when M is Cl, Y is OK and n is equal to 1.

4. Process according to claim 3 wherein R₁ denotes H, F, an alkyl group or a fluoroalkyl group; preferably F, an alkyl group or a fluoroalkyl group; more preferably F or a fluoroalkyl group; still more preferably a C₁-C₄ fluoro or perfluoroalkyl group and even more preferably CF₃.

5. Process according to claim 3 or 4, wherein, said halogen atom in connection with Y and/or M' is chlorine.

6. Process according to any of claims 3 to 5, wherein said salt of the cyanide compound is of formula M'^{q+}(⁻C≡N)_{q} wherein M'^{q+} is selected from metal and onium cations and q denotes the valency of said cation; preferably M'^{q+} is selected from alkali metal, alkaline earth metal, transition metal, phosphonium and ammonium cations.

7. Process according to any of claims 3 to 6, wherein the reaction is performed in a solvent.

8. Process according to any of claims 3 to 7, wherein the reaction is performed by means of a catalyst.

9. Process according to any of claims 3 to 8, wherein compound of formula (i₅) wherein n is equal to 1 and Y is a halogen atom is obtained by a reaction step between a compound of formula (i₆) 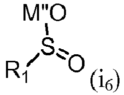
wherein R₁ denotes H, a halogen atom, an alkyl group or a halogenoalkyl group and M" denotes an alkali metal,
and a compound (vi) of formula X₅-S(O)-X₆, wherein X₅ and X₆, which can be the same or different, denote respectively a halogen atom different from R₁.

10. Process according to any of claims 3 to 9, further comprising at least one step of :
- recovering and/or purifying compound (VI), preferably by at least one filtration and/or distillation; and/or
- separating part or all of the unreacted compound (i₅), (iv), (iv') (iv") and/or the cyanide compound M'CN and in recycling these compounds in the process.

11. Electrolyte composition, comprising at least one electrolyte salt and at least one compound of formula (VI) according to any of claims 1 or 2 or obtained according to any of claims 3 to 10.

12. Electrolyte composition according to claim 11, further comprising at least one of the following compounds:
- compound of formula (VIII) 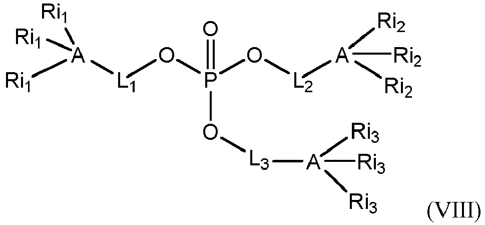
wherein A is Si or C;
L₁, L₂ and L₃, which can be the same or different, independently denote a simple bond, a substituted or unsubstituted, linear or branched, saturated or unsaturated, C₁-C₄ alkylene, C₃-C₁₂ cycloalkylene, C₃-C₁₂ arylene, or C₄-C₁₆ arylenealkylene group, optionally comprising at least one heteroatom selected from N, O and S;
Ri₁, Ri₂ and Ri₃, which can be the same or different, independently denote H, a halogen atom, a linear or branched C₁-C₈ alkyl, C₂-C₈ alkenyl, C₂-C₈ alkynyl, or C₆-C₁₀ aryl group, optionally comprising at least one substituent selected from a halogens, hydroxyl, alkoxy, carbonyl, and carboxyl groups, and/or optionnaly comprising at least one heteroatom selected from N, O and S;
- compound of formula (IX) 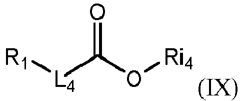
wherein R₁ denotes H, a halogen atom, an alkyl group or a fluoroalkyl group;
L₄ denotes a simple bond or a substituted or unsubstituted, linear or branched, saturated or unsaturated, C₁-C₄ alkylene, C₃-C₁₂ cycloalkylene, C₃-C₁₂ arylene, or C₄-C₁₆ arylenealkylene group, optionnaly comprising at least one heteroatom selected from N, O and S;
Ri₄ denotes H, a linear or branched C₁-C₈ alkyl, C₂-C₈ alkenyl, C₂-C₈ alkynyl, or C₆-C₁₀ aryl group, optionally comprising at least one substituent selected from a halogens, hydroxyl, alkoxy, carbonyl, and carboxyl groups, and/or optionally comprising at least one heteroatom selected from N, O and S;
- compound of formula (X) 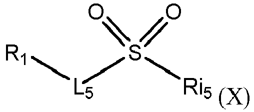
wherein R₁ denotes H, a halogen atom, an alkyl group or a halogenoalkyl group;
L₅ denotes a simple bond or a substituted or unsubstituted, linear or branched, saturated or unsaturated, C₁-C₄ alkylene, C₃-C₁₂ cycloalkylene, C₃-C₁₂ arylene, or C₄-C₁₆ arylenealkylene group, optionnaly comprising at least one heteroatom selected from N, O and S;
Ri₅ denotes H, a linear or branched C₁-C₈ alkyl, C₂-C₈ alkenyl, C₂-C₈ alkynyl, or C₆-C₁₀ aryl group, optionally comprising at least one substituent selected from a halogens, hydroxyl, alkoxy, carbonyl, and carboxyl groups, and/or optionally comprising at least one heteroatom selected from N, O and S;
- compound of formula (XI) 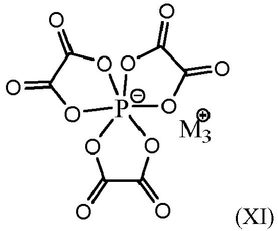 wherein M₃ is H, a metal or N(Ri₆Ri₇Ri₈Ri₉), wherein Ri₆, Ri₇, Ri₈ and Ri₉, which can be the same or different, independently denote H or a C₁-C₁₂ alkyl group.
- compound of formula (XII) 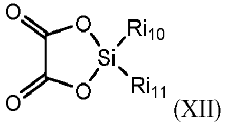 wherein Ri₁₀ and Ri₁₁, which can be the same or different, independently denote H, a C₁-C₈ alkyl, C₂-C₈ alkenyl, C₂-C₈ alkynyl, or C₆-C₁₀ aryl group, optionally comprising at least one substituent selected from halogens, hydroxyl, alkoxy, carbonyl, and carboxyl groups;
- compound of formula (XIII) 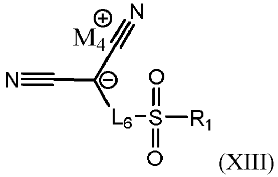
wherein R₁ denotes H, a halogen atom, an alkyl group or a halogenoalkyl group;
L₆ is a simple bond or a substituted or unsubstituted, linear or branched, saturated or unsaturated, C₁-C₄ alkylene, C₃-C₁₂ cycloalkylene, C₃-C₁₂ arylene, or C₄-C₁₆ arylenealkylene group, optionally comprising at least one heteroatom selected from N, O and S;
M₄ is H, a metal or N(Ri₆Ri₇Ri₈Ri₉), wherein Ri₆, Ri₇, Ri₈ and Ri₉, which can be the same or different, independently denote H or a C₁-C₁₂ alkyl group;
- compound of formula (XIV) 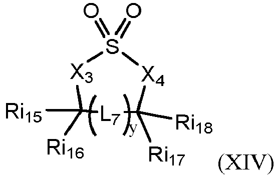
wherein X₃ and X₄, which can be the same or different, are independently selected from:
▪ NRi₁₂ where Ri₁₂ is H, a C₁-C₄ alkyl group, a C₁-C₄ alkenyl group or a C₁-C₄ alkynyl group;
▪ CRi₁₃Ri₁₄ where Ri₁₃ and Ri₁₄, which can be the same or different, are independently H, a C₁-C₄ alkyl group, a C₁-C₄ alkenyl group or a C₁-C₄ alkynyl group;
▪ and O;
Ri₁₅, Ri₁₆, Ri₁₇ and Ri₁₈, which can be the same or different, are independently selected from H, a halogen atom, a C₁-C₄ alkyl group and a C₁-C₄ halogenoalkyl group;
y is an integer ranging from 0 to 2 such as (L₇)₀ stands for a simple bond and when y is 1 or 2, L₇ stands for a CRi₁₉Ri₂₀ group where Ri₁₉ and Ri₂₀, which can be the same or different, are H, a C₁-C₄ alkyl group, a C₁-C₄ alkenyl group or a C₁-C₄ alkynyl group;
- compound of formula (XV) 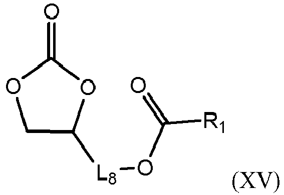 wherein R₁ denotes H, a halogen atom, a C₁-C₄ alkyl group or a C₁-C₄ halogenoalkyl group; L8 is a simple bond or a substituted or unsubstituted, linear or branched, saturated or unsaturated, C₁-C₄ alkylene, C₃-C₁₂ cycloalkylene, C₃-C₁₂ arylene, or C₄-C₁₆ arylenealkylene group, optionnaly comprising at least one heteroatom selected from N, O and S;
- compound of formula (XVI) 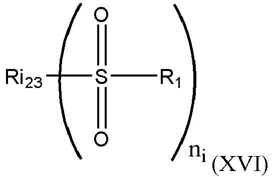
wherein R₁ denotes H, a halogen atom, a C₁-C₄ alkyl group or a C₁-C₄ halogenoalkyl group; nᵢ denotes an integer greater than or equal to 2,
Ri₂₃ denotes a nᵢ-valent linkage group comprising at least one carbon atom and atoms selected from C, H, a halogen atom and O, and the S atom of the -SO₂R₁ group is bound to a carbon atom of the Ri₂₃ group;
- compound of formula (XVII) 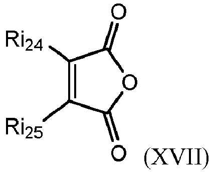 wherein Ri₂₄ and Ri₂₅, which can be the same or different, are independently selected from H, a halogen atom, a C₁-C₄ alkyl group, and a C₁-C₄ halogenoalkyl group;
- and mixtures thereof.

13. Electrolyte composition according to claim 11 or 12 further comprising:
- at least one non-fluorinated cyclic carbonate, preferably selected from ethylene carbonate, propylene carbonate, vinylene carbonate, ethyl propyl vinylene carbonate, vinyl ethylene carbonate, dimethylvinylene carbonate, and mixtures thereof; and/or
- at least one non-fluorinated acyclic carbonate, preferably selected from ethyl methyl carbonate, dimethyl carbonate, diethyl carbonate, di-tert-butyl carbonate, dipropyl carbonate, di-tert-butyl carbonate, dipropyl carbonate, methyl propyl carbonate, methyl butyl carbonate, ethyl butyl carbonate, propyl butyl carbonate, dibutyl carbonate, and mixtures thereof; and/or
- at least one fluorinated cyclic carbonate, preferably selected from 4-fluoro-1,3-dioxolan-2-one; 4-fluoro-4 -methyl-1,3-dioxolan-2-one; 4-fluoro-5-methyl-1,3-dioxolan-2-one; 4-fluoro-4,5-dimethyl-1,3-dioxolan-2-one; 4,5-difluoro-1,3-dioxolan-2-one; 4,5-difluoro-4-methyl-1,3-dioxolan-2-one; 4,5-difluoro-4,5-dimethyl-1,3-dioxolan-2-one; 4,4-difluoro-1,3-dioxolan-2-one; 4,4,5-trifluoro-1,3-dioxolan-2-one; 4,4,5,5-tetrafluoro-1,3-dioxolan-2-one; and mixtures thereof; being preferably 4-fluoro-1,3-dioxolan-2-one.

14. Electrolyte composition according to any of claims 11 to 13, further comprising at least one compound selected from:
- a fluorinated acyclic carboxylic acid ester represented by the formula:
R₁₃-COO-R₁₄,
- a fluorinated acyclic carbonate represented by the formula:
R₁₅-OCOO-R₁₆,
- a fluorinated acyclic ether represented by the formula:
R₁₇-O-R₁₈,
- and mixtures thereof,
wherein
i) R₁₃ is H, an alkyl group, or a fluoroalkyl group;
ii) R₁₅ and R₁₇ is each independently a fluoroalkyl group and can be either the same as or different from each other;
iii) R₁₄, R₁₆, and R₁₈ is each independently an alkyl group or a fluoroalkyl group and can be either the same as or different from each other;
iv) either or both of R₁₃ and R₁₄ comprises fluorine; and
v) R₁₃ and R₁₄, R₁₅ and R₁₆, R₁₇ and R₁₈, each taken as a pair, comprise at least two carbon atoms but not more than seven carbon atoms.

15. Electrolyte composition according to any of claims 11 to 14, wherein said electrolyte salt is a lithium salt, preferably selected from hexafluorophosphate (LiPF₆), lithium bis(trifluoromethyl)tetrafluorophosphate (LiPF₄(CF₃)₂), lithium bis(pentafluoroethyl)tetrafluorophosphate (LiPF₄(C₂F₅)₂), lithium tris(pentafluoroethyl)trifluorophosphate (LiPF₃(C₂F₅)₃), lithium bis(trifluoromethanesulfonyl)imide (LiN(CF₃SO₂)₂), lithium bis(perfluoroethanesulfonyl)imide LiN(C₂F₅SO₂)₂, LiN(C₂F₅SO₃)₂, lithium (fluorosulfonyl) (nonafluorobutanesulfonyl)imide, lithium bis(fluorosulfonyl)imide, lithium tetrafluoroborate, lithium perchlorate, lithium hexafluoroarsenate, lithium hexafluoroantimonate, lithium tetrachloroaluminate, LiAlO₄, lithium trifluoromethanesulfonate, lithium nonafluorobutanesulfonate, lithium tris(trifluoromethanesulfonyl)methide, lithium bis(oxalato)borate, lithium difluoro(oxalato)borate, Li₂B1₂F₁₂₋ₓHₓ where x is an integer equal to 0 to 8, and mixtures of lithium fluoride and anion receptors such as B(OC₆F₅)₃, being preferably selected from lithium hexafluorophosphate, lithium bis(fluorosulfonyl)imide and lithium bis(trifluoromethanesulfonyl)imide, and preferably being hexafluorophosphate.
